# EUROPEAN PATENT APPLICATION

(11) **EP 4 692 087 A1**
(43) Date of publication of application: **11.02.2026**
(21) Application number: 24784285.9
(22) Date of filing: 02.04.2024
(51) Int. Cl.: C07D 473/34, A61K 31/52, A61P 25/16

(54) **NON-COVALENT DIMER CATION OF PURINE/PYRIMIDINE AND AMINO ACID, SALT THEREOF, PREPARATION METHOD THEREFOR, AND USE THEREOF**

(30) Priority: 03.04.2023 CN 202310344800
(71) Applicant: Suzhou Protonation Chain Biotechnology Co., Ltd, Suzhou, Jiangsu 215011 (CN)
(72) Inventor: ZHAO, Zizhen, Suzhou, Jiangsu 215011 (CN); WANG, Zhigang, Suzhou, Jiangsu 215011 (CN)
(74) Representative: Schulz Junghans Patentanwälte PartGmbB
(86) International application number: PCT/CN2024/085549
(87) International publication number: WO 2024/208202

(57) **Abstract**

Provided is a non-covalent dimer cation of the present application, comprising or consisting of a cation of formula [⁺Q¹-H···Q²], a tautomer thereof, or a stereoisomer thereof. Provided is a salt of the present application, including the cation of the present application and a first anion. Provided is use of the cation of the present application or the salt of the present application, for example, use in the preparation of a medicament for promoting cell repair, cell proliferation, or wound repair.

## Description

### FIELD

The present application relates to the field of biomedicines, and particularly, to a non-covalent dimer cation consisting of purine or derivatives thereof and/or pyrimidine or derivatives thereof and amino acid, a salt thereof, a preparation method thereof, and use thereof in cell repair, cell proliferation, wound repair, or other aspects.

### BACKGROUND

The cell repair ability plays an important role in living organisms. Many factors may cause cell damage, which in turn leads to tissue and organ damages, thereby resulting in diseases. In this case, the cell self-repair ability is particularly important. A number of studies have shown that young cells have a stronger ability and greater inclination to self-repair than aged cells. That is, young cells are more inclined to repair themselves rather than waiting for immune cells to repair. In addition, young cells have a stronger repair ability.

The cell repair ability also varies significantly according to different cells. Cells of various glandular organs, such as liver, pancreas, endocrine glands, sweat glands, sebaceous glands, and renal tubular epithelial cells, exhibit strong repair ability when they are damaged internally or externally. Cells with weak or no repair ability, such as central nervous cells and ganglion cells, are extremely difficult to restore their original functions after being damaged. Cardiac muscle cells are replaced by fibrous connective tissues after being damaged and thus very difficult to restore their original structures and functions due to extremely weak repair ability.

The occurrence of many diseases is also closely related to the decline or deficiency of the cell repair ability, and the significantly increased probability of certain diseases in the elderly and those exposed to risk is caused by the decline or deficiency of cell repair levels in their bodies. For example, neurodegenerative diseases such as Parkinson's disease and Alzheimer's disease that are suffered by the elderly, gastric mucosal damage, gastric ulcers and the like that occur in people who are heavy drinkers over a long period of time, or some organ diseases caused by excessively large pressure of organ functions, are all caused by the insufficient cell repair ability.

Studies have shown that in the vast majority of damaged cells, there is usually a high level of oxidative stress. That is, when the cells are damaged, they reflect an abnormal state, which is caused by damages or damage repair. In addition, a high ROS level will occur, thereby inhibiting the cell self-repair function, and further exacerbating the diseases. Therefore, reducing an intracellular ROS level is also an effective means to restore the cell repair ability, but this method does not work directly, resulting in poor effect.

There are many factors that affect the cell repair ability, including the types, degrees, and extents of damages, the age of individuals, the nutritional statuses of organisms, or the effects of drugs. However, the treatment of related diseases in the current clinical stage still focuses on the "targeted" treatment of the diseases, while ignoring the cell self-repair ability. Therefore, the progress in the treatment means of some diseases is slow, and due to side effects of some drugs, the phenomenon of prioritizing one aspect while neglecting another often occurs.

Cell proliferation, as one of the important physiological functions of living cells, is an important life characteristic of living organisms. The cell proliferation is the basis for the growth, development, reproduction, and heredity of living organisms. It is also an important life characteristic of living organisms. Human cells proliferate through division to replenish aging or dead cells in the body.

Cell proliferation is a complex and vital life activity. The development of a fertilized egg into an independent individual involves countless rounds of cell proliferation. Each organ also undergoes regulated cell proliferation all the time to maintain its physiological life activities. When invaded by pathogens, relevant immune cells proliferate to protect the individual's health. When an individual suffers physical wounds, the relevant cells will also start to proliferate to repair tissue or organ damages.

Wound refers to the destruction of human tissues or organs caused by mechanical factors. It is extremely common, occurring not only on a large scale during wartime but also in peacetime. Wounds are of a broad concept, including: cuts, stabs, bruises, and sprains. Due to the rapid development of industries, agriculture, transportation, and sports, the number of wounds caused by various accidents is increasing day by day. The wounds have high incidence and broad influences. Due to the maturity and popularization of internal medicine and surgical procedures, surgical wounds, as a type of wounds, also frequently occur in people's daily lives.

Wound healing refers to a series of complex pathological and physiological processes that occur during external injuries or other diseases, where local tissues are repaired by proliferation or regeneration after tissue defects. After the occurrence of a wound, the body undergoes a highly coordinated healing process, including the complex interplay of various cells, cytokines, and extracellular matrices. The cells participating in skin wound healing mainly include various inflammatory cells and tissue repair cells. The inflammatory cells include neutrophils, macrophages, etc., and the tissue repair cells mainly include vascular endothelial cells, epidermal cells, fibroblasts, and nerve cells. These series of cells are indispensable for achieving their respective functions through cell proliferation and promoting the healing of wound surfaces.

Currently, in clinical treatment, the focus is mainly on performing sterilization and disinfection on wounds for a cleaning purpose. However, as for the specific repair work, it still relies on the body's own coordinated repair ability. Elderly people have a weak tissue cell proliferation ability, which leads to slow wound healing, high infection risk, and interference with normal life. In severe cases, the inflammations caused by infections can even threaten the life. However, when the proliferation rate of healed tissue cells is slow, a relatively hypertrophic connective tissue will be formed. This connective tissue is difficult to integrate with the original skin, thus presenting as a scar, which affects the appearance. Therefore, some studies suggest that accelerating the wound healing process may facilitate reducing the formation of scars.

### SUMMARY

A non-covalent dimer cation is provided, comprising or consisting of a cation of formula [⁺Q¹-H···Q²], a tautomer thereof, or a stereoisomer thereof, wherein a bond "···" in formula [⁺Q¹-H···Q²] is a non-covalent bond. One of Q¹ and Q² is a first moiety, and the other is a second moiety. The first moiety is selected from a group consisting of formula (R), formula (Y), and dihydro derivatives thereof, and the second moiety is formula (A),

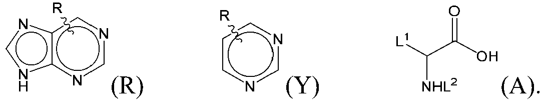

R is selected from a group consisting of no group, -H, -CH₃, =CH₂, -NH₂, =NH, -OH, =O, -F, β-D-ribofuranosyl, and 2-deoxy-β-D-ribofuranosyl. -L² is H, and -L¹ is selected from a group consisting of hydrogen, methyl, hydroxymethyl, mercaptomethyl, selenomethyl, aminomethyl, carboxymethyl, carbamoylmethyl, methylthiomethyl, guanidinomethyl, ethyl, 1-hydroxyethyl, 2-hydroxyethyl, 1-mercaptoethyl, 2-mercaptoethyl, 1-aminoethyl, 2-aminoethyl, 1-carboxyethyl, 2-carboxyethyl, 1-carbamoylethyl, 2-carbamoylethyl, 1-methylthioethyl, 2-methylthioethyl, 1-guanidinoethyl, 2-guanidinoethyl, n-propyl, isopropyl, 1-hydroxypropyl, 2-hydroxypropyl, 3-hydroxypropyl, 1-mercaptopropyl, 2-mercaptopropyl, 3-mercaptopropyl, 1-aminopropyl, 2-aminopropyl, 3-aminopropyl, 1-carboxypropyl, 2-carboxypropyl, 3-carboxypropyl, 1-carbamoylpropyl, 2-carbamoylpropyl, 3-carbamoylpropyl, 1-methylthiopropyl, 2-methylthiopropyl, 3-methylthiopropyl, 1-guanidinopropyl, 2-guanidinopropyl, 3-guanidinopropyl, n-butyl, isobutyl, sec-butyl, tert-butyl, 4-aminobutyl, 1-hydroxybutyl, 2-hydroxybutyl, 3-hydroxybutyl, 4-hydroxybutyl, 1-mercaptobutyl, 2-mercaptobutyl, 3-mercaptobutyl, 4-mercaptobutyl, 1-aminobutyl, 2-aminobutyl, 3-aminobutyl, 4-aminobutyl, 1-carboxybutyl, 2-carboxybutyl, 3-carboxybutyl, 4-carboxybutyl, 1-carbamoylbutyl, 2-carbamoylbutyl, 3-carbamoylbutyl, 4-carbamoylbutyl, 1-methylthiobutyl, 2-methylthiobutyl, 3-methylthiobutyl, 4-methylthiobutyl, 1-guanidinobutyl, 2-guanidinobutyl, 3-guanidinobutyl, 4-guanidinobutyl, benzyl, 2-hydroxybenzyl, 3-hydroxybenzyl, 4-hydroxybenzyl, (pyrrol-4-yl)methyl, (indol-3-yl)methyl, and (3-methyl-3,4-dihydropyrrole-2-carbonyl)aminobutyl; or -L¹ and -L² together form a group selected from a group consisting of propane-1,3-diyl, butane-1,4-diyl, and pentane-1,5-diyl.

A salt of the present application is provided, comprising the cation of the present application, and a first anion comprising at least one selected from a group consisting of a fluoride ion, a chloride ion, a bromide ion, an iodide ion, a sulfide ion, a nitrate ion, a sulfate ion, a sulfite ion, a thiosulfate ion, a persulfate ion, a selenate ion, a phosphate ion, a carbonate ion, a hexafluorophosphate ion, a hexafluorosilicate ion, an acetate ion, a succinate ion, a sulfonate ion, a benzoate ion, and a polyphosphate ion.

A method of preparing the cation of the present application or the salt of the present application is provided, comprising: contacting a first reactant with a first acid to obtain a first acid salt of the first reactant; contacting the first acid salt of the first reactant with a first salt comprising a first anion in a first acidic environment, to obtain a first solution comprising a protonated first acid salt cation of the first reactant and the first anion; adding a second reactant to the first solution in a second acidic environment, to obtain a second solution or a first intermediate; and adding a first base to the second solution or contacting the first base with the first intermediate, wherein the first reactant comprises a compound of formula Q¹, and the second reactant comprises a compound of formula Q².

Use of the cation of the present application or the salt of the present application in preparation of a medicament, and particularly, in antioxidation, preparation of an antioxidant, preparation of a medicament for inhibiting, reducing, or reversing oxidative stress in human or animal cells or for inhibiting, reducing, or scavenging oxygen species in human or animal bodies, or preparation of a medicament for treating an oxidative stress- or oxygen species-associated disease or symptom is provided. A method for preparing a medicament is provided, comprising preparing the cation of the present application or the salt of the present application.

Use of the cation of the present application or the salt of the present application in preparation of a medicament, and particularly, in promotion of cell proliferation, or preparation of a medicament for promoting cell proliferation or treating, alleviating, or repairing wounds is provided. A method for preparing a medicament is provided, comprising preparing the cation of the present application or the salt of the present application.

### DETAILED DESCRIPTION

As used herein, the singular term refers to one or more. For example, "element" or "an element" refers to one or more elements. As used herein, the term "a plurality of" refers to at least two.

As used herein, the term "about" refers to approximately, in the range of about or near. When used in combination with a value range, the term "about" modifies the range by extending the limit above or below the value provided. Generally, the term "about" is used herein to indicate a variation of ±10% around the provided value. In one aspect, the term "about" refers to adding or subtracting 20% of the value it modifies. For example, "about 50%" refers to a range of 45%-55%. Herein, a value range defined by endpoints includes all integers and fractions within the range (for example "1 to 5", including but not limited to, 1, 1.5, 2, 2.75, 3, 3.90, 4, and 5). It should also be understood that all the integers and fractions are defined by the term "about".

As used herein, the term "include", "comprise", or "have", as a non-exclusive or open-ended term, is intended to mean that a combination (such as a device, a composition, or a method) includes listed elements (such as units of the device, components of the composition, or substantive steps of the method), but does not exclude other elements. As used herein, the term "essentially consist of ..." means, when used for defining a composition or a method, excluding other elements that have any substantive effect on the combination of the stated objectives, but not excluding other elements that do not essentially affect the basic and novel features of the present application. As used herein, unless otherwise described, the term "consist of ..." means excluding the combination of other elements (units, components, substantive steps, or the like), and not intended to exclude trace amounts of unavoidable impurities. Embodiments defined by each of these connection terms fall within the scope of the present application. As a specific embodiment, disclosure of a technical solution including the term "include", "comprise", or "have" should also be regarded as simultaneously disclosing a corresponding technical solution including the term "essentially consisting of ..." or "consisting of ...".

As used herein, the term "and/or" refers to and covers any or all possible combinations of one or more associated listed items. When used in a list of two or more items, the term "and/or" means that any one of the listed items may be contained alone, or any combination of two or more of the listed items may be contained. For example, if a group, combination, composition, or the like is described as including (or containing) components A, B, C, and/or D, the composition may contain A alone; B alone; C alone; D alone; a combination of A and B; a combination of A and C; a combination of A and D; a combination of B and C; a combination of B and D; a combination of C and D; a combination of A, B, and C; a combination of A, B, and D; a combination of A, C, and D; a combination of B, C, and D; or a combination of A, B, C, and D.

As used herein, the compound or ion of the present application comprises a plurality of variable groups. A person skilled in the art will recognize that combinations of the groups that are contemplated by the present application are chemically allowed combinations of compounds or ions.

As used herein, the stereochemistry of a chiral center may be defined according to the conventions of a person skilled in the art. That is, the wedged bond " 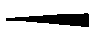 " is used for indicating a group in front of the plane of the paper (oriented towards the viewer), and the dashed bond " 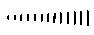 " is used for indicating a group behind the plane of the paper (oriented away from the viewer). It may be understood that such representations are used for indicating a specific single stereoisomer of groups represented by chemical structures herein. Any bond that is not specifically represented by the wedged bond or the hashed bond herein shall be regarded as not specifically indicating whether the bond is in front of the plane of the paper, behind the plane of the paper, or located in the plane of the paper. However, it does not exclude that the bond is in front of or behind the plane of the paper if chemically allowed.

As used herein, the term "isomer" refers to compounds with the same molecular formula and different bonding nature or order of atoms or different arrangements of atoms in space. The term "stereoisomer" refers to isomers with different arrangements of atoms in space. The term "enantiomer" refers to stereoisomers with one or more asymmetric centers that are non-superimposable mirror images of each other. The term "diastereoisomer" refers to stereoisomers that have opposite configurations of one or more asymmetric centers and that do not belong to enantiomers. When a compound has an asymmetric center, for example, if a carbon atom is bonded to four different groups, a pair of enantiomers may exist. A configuration of an enantiomer may be characterized and designated as an *R*-configuration or an *S*-configuration through an absolute configuration of one or more asymmetric centers of the enantiomer, or the enantiomer is designated as dextrorotatory or levorotatory based on the manner in which the molecule rotates the plane of polarized light. A chiral compound may exist in the form of an enantiomer alone or a mixture of enantiomers, for example, in the form of a racemic mixture. The compound of the present application may contain an asymmetric center or chiral center, and exist in the form of different stereoisomers. It should be regarded that all stereoisomers of the compound of the present application comprise, but are not limited to, a diastereoisomer, an enantiomer, an atropisomer, and a mixture thereof, such as a racemic mixture, which form part of the present application.

As used herein, the term "dimer" generally refers to a complex formed by two identical or different molecules or ions through an interaction, particularly a non-covalent interaction. As used herein, the term "n-mer" generally refers to a complex formed by n molecules or ions through interactions, particularly non-covalent interactions. As used herein, the term "covalent bond" refers to any bond that contains or involves electron sharing. Non-limiting examples of covalent bonds comprise, but are not limited to, a peptide bond, a glycosidic bond, an ester bond, and a phosphodiester bond, and particularly comprise a coordinate-covalent bond. As used herein, the term "non-covalent bond" comprises any bond or interaction between two or more moieties that do not contain or involve electron sharing. Non-limiting examples of non-covalent bonds or interactions comprise, but are not limited to, static electricity, π-π effects, the van der Waals force, a hydrogen bond, and the hydrophobic effect, particularly the hydrogen bond. As used herein, the term "bonding" comprises at least covalent bonds and non-covalent bonds.

As used herein, any amino acid with chirality shall be regarded as at least referring to L-amino acid in a case that the chirality is not explicitly described. However, it does not explicitly exclude relevant embodiments of β-D-amino acid.

As used herein, particularly, the term "disclose or comprise" as described below comprises, but is not limited to, the disclosure of the specification (such as, examples) or claims of the present application, and comprises, but is not limited to, the scope of protection of the claims of the present application, or any one or more embodiments (such as, embodiments defined by the term "in an embodiment" or "in some embodiments" herein).

To further describe the technical means adopted by the present application to achieve the intended objective and effects, a detailed description of specific embodiments, structures, features, and effects of the present application is provided below with reference to preferred examples.

### Non-covalent dimer cation of the present application

In an aspect, the present application provides a non-covalent dimer cation.

The present application provides a non-covalent dimer cation, comprising or consisting of a cation of formula [⁺Q¹-H···Q²], a tautomer thereof, or a stereoisomer thereof. A bond "···" in formula [⁺Q¹-H···Q²] is a non-covalent bond. One of a Q¹ moiety and a Q² moiety is a first moiety. The Q¹ moiety and the Q² moiety are each independently selected from a group consisting of formula (R), formula (Y), and dihydro derivatives thereof, and particularly, each independently selected from a group consisting of formula (R) and formula (Y). The other of the Q¹ moiety and the Q² moiety is a second moiety and is formula (A):

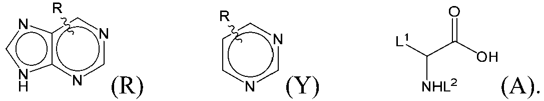

The non-covalent bond, *i.e.*, the bond "···" in formula [⁺Q¹-H···Q²] (represented by "-------" in structural formulae of the examples), shall be regarded as including all chemically allowed non-covalent bonds that enable the non-covalent dimer cation of the present application to exist stably in its salts, or consisting of the chemically allowed non-covalent bonds.

According to the understanding of a person skilled in the art, the non-covalent bond may be understood as a hydrogen bond. However, through experiments, the applicant has confirmed that the non-covalent bond exists stably in the general environment, for example, in the form of a salt. Particularly, the non-covalent bond can remain stable without being broken at least during determination by mass spectroscopy. That is, the non-covalent dimer cation of the present application can be kept intact during determination by mass spectroscopy, and accordingly an ion peak corresponding to an m/z value of the non-covalent dimer cation of the present application is generated. That is, it should be understood that properties of some hydrogen bonds may be applicable to the non-covalent bond. However, the non-covalent bond does not necessarily comply with all understandings of properties of hydrogen bonds by a person skilled in the art, especially in terms of bond energy. In some embodiments, the non-covalent bond is a bond with bond energy stronger than that of a general hydrogen bond, such as a bond with bond energy greater than 8 kJ/mol.

" 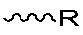 " marked in the structural formula indicates that a substituent R may be formed on any constituent atom (member) of the structural formula. Specifically,
- " 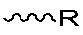 " marked in formula (R) indicates that one or more identical or different substituents R are formed or separately formed on one or more of the atoms at position 1, position 2, position 3, position 6, position 7, position 8, or position 9 of the purine ring.
- " 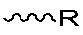 " marked in formula (Y) indicates that one or more identical or different substituents R are formed or separately formed on one or more of the atoms at position 1, position 2, position 3, position 4, position 5, or position 6 of the pyrimidine ring.

In some embodiments, the first moiety is nucleobase, nucleoside, or deoxynucleoside. In some embodiments, the first moiety is nucleobase. In some embodiments, the first moiety is nucleoside. In some embodiments, the first moiety is deoxynucleoside.

In some embodiments, R is selected from a group consisting of no group, -X¹, and =X². -X¹ is selected from a group consisting of -H, -CH₃, -NH₂, -OH, -F, β-D-ribofuranosyl, and 2-deoxy-β-D-ribofuranosyl, and =X² is selected from a group consisting of =CH₂, =NH, and =O. In some embodiments, R is selected from a group consisting of -X¹ and =X². In some embodiments, R is -X¹. In some embodiments, R is =X². In some embodiments, R is selected from a group consisting of -H, -CH₃, =CH₂, -NH₂, =NH, -OH, =O, -F, β-D-ribofuranosyl, and 2-deoxy-β-D-ribofuranosyl. In some embodiments, R is -H. In some embodiments, R is -CH₃. In some embodiments, R is =CH₂. In some embodiments, R is -NH₂. In some embodiments, R is =NH. In some embodiments, R is -OH. In some embodiments, R is =O. In some embodiments, R is -F. In some embodiments, R is β-D-ribofuranosyl. In some embodiments, R is 2-deoxy-β-D-ribofuranosyl.

As used herein, when a substituent is referred to as a "no group", it indicates that the atom to which the substituent is attached (e.g., an N atom) is not attached to other groups not shown in the structural formula if it is electrically neutral. However, it is not intended to exclude that the atom is ionized, such as being protonated or forming a quaternary ammonium salt. For example, when a substituent -Z on the " 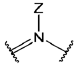 " moiety is referred to as a "no group", it indicates that the N atom to which the substituent -Z is attached is not attached to other groups not shown in the structural formula, that is, the moiety is further defined as a " 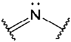 " moiety in the case of electrical neutrality. However, it is not intended to exclude, for example, the " 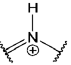 " moiety in the case of protonation. The no group may be a lone pair of electrons, which may be protonated to cause the resident atom to form an ion.

In some embodiments, formula (R) is formula (R'), and/or formula (Y) is formula (Y').

In some embodiments, the first moiety is selected from a group consisting of formula (R'), formula (Y'), and dihydro derivatives thereof, and particularly, selected from a group consisting of formula (R') and formula (Y'),

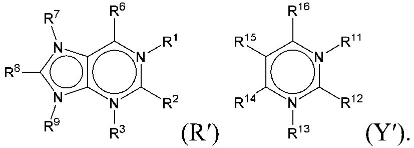

R¹, R², R³, R⁶, R⁷, R⁸, R⁹, R¹¹, R¹², R¹³, R¹⁴, R¹⁵, and R¹⁶ are each independently selected from a group consisting of no group, -X¹, and =X², and particularly, selected from the group consisting of no group, -H, -CH₃, =CH₂, -NH₂, =NH, -OH, =O, -F, β-D-ribofuranosyl, and 2-deoxy-β-D-ribofuranosyl. When the Q¹ moiety and the Q² moiety are both selected from formula (R'), R¹, R², R³, R⁶, R⁷, R⁸, and R⁹ of the Q¹ moiety may be the same as or different from R¹, R², R³, R⁶, R⁷, R⁸, and R⁹ of the corresponding Q² moiety. When the Q¹ moiety and the Q² moiety are both selected from formula (Y'), R¹¹, R¹², R¹³, R¹⁴, R¹⁵, and R¹⁶ of the Q¹ moiety may be the same as or different from R¹¹, R¹², R¹³, R¹⁴, R¹⁵, and R¹⁶ of the corresponding Q² moiety.

In some embodiments, R¹ is no group, and R² or R⁶ is -X¹; or R¹ is -X¹, and R² or R⁶ is =X². In some embodiments, R¹ is no group, and R² is -X¹; or R¹ is -X¹, and R² is =X². In some embodiments, R¹ is no group, and R⁶ is -X¹; or R¹ is -X¹, and R⁶ is =X². In some embodiments, R¹ or R³ is no group, and R² is -X¹; or R¹ or R³ is -X¹, and R² is =X². In some embodiments, R¹ is no group, and R² is -X¹; or R¹ is -X¹, and R² is =X². In some embodiments, R³ is no group, and R² is -X¹; or R³ is -X¹, and R² is =X². In some embodiments, R⁷ is no group, R⁸ is -X¹, and R⁹ is -X¹. In some embodiments, R⁷ is -X¹, R⁸ is -X¹, and R⁹ is no group. In some embodiments, R⁷ is -X¹, R⁸ is =X², and R⁹ is -X¹. In some embodiments, R¹¹ is no group, and R¹² or R¹⁶ is -X¹; or R¹¹ is -X¹, and R¹² or R¹⁶ is =X². In some embodiments, R¹¹ is no group, and R¹² is -X¹; or R¹¹ is - X¹, and R¹² is =X². In some embodiments, R¹¹ is no group, and R¹⁶ is -X¹; or R¹¹ is -X¹, and R¹⁶ is =X². In some embodiments, R¹¹ or R¹³ is no group, and R¹² is -X¹; or R¹¹ or R¹³ is -X¹, and R¹² is =X². In some embodiments, R¹¹ is no group, and R¹² is -X¹; or R¹¹ is -X¹, and R¹² is =X². In some embodiments, R¹³ is no group, and R¹² is -X¹; or R¹³ is -X¹, and R¹² is =X². In some embodiments, R¹³ is no group, and R¹² or R¹⁴ is -X¹; or R¹³ is -X¹, and R¹² or R¹⁴ is =X². In some embodiments, R¹³ is no group, and R¹² is -X¹; or R¹³ is -X¹, and R¹² is =X². In some embodiments, R¹³ is no group, and R¹⁴ is -X¹; or R¹³ is -X¹, and R¹⁴ is =X².

In some embodiments, formula (R) is formula (Ra) or formula (Rb), and/or formula (Y) is formula (Ya) or formula (Yb).

In some embodiments, the first moiety is selected from a group consisting of formula (Ra), formula (Rb), formula (Ya), and formula (Yb),

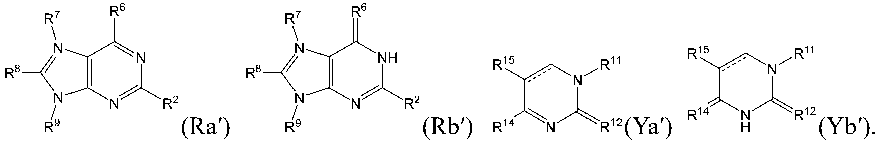

-R², -R⁸, -R¹¹, and -R¹⁵ are -X¹, and particularly, each independently selected from a group consisting of -H, -CH₃, -NH₂, -OH, -F, β-D-ribofuranosyl, and 2-deoxy-β-D-ribofuranosyl. =R¹² is =X², and particularly, selected from a group consisting of =CH₂, =NH, and =O. R⁶ is -X¹ in formula (Ra') and =X² in formula (Rb'). Particularly, in formula (Ra'), -R⁶ is selected from the group consisting of -H, -CH₃, -NH₂, -OH, -F, β-D-ribofuranosyl, and 2-deoxy-β-D-ribofuranosyl, and in formula (Rb'), =R⁶ is selected from the group consisting of =CH₂, =NH, and =O. R¹⁴ is - X¹ in formula (Ya') and =X² in formula (Yb'). Particularly, in formula (Ya'), -R¹⁴ is selected from the group consisting of -H, -CH₃, -NH₂, -OH, -F, β-D-ribofuranosyl, and 2-deoxy-β-D-ribofuranosyl, and in formula (Yb'), =R¹⁴ is selected from the group consisting of =CH₂, =NH, and =O. In addition, -R⁷ is no group, and -R⁹ is -X¹. Particularly, they are each independently selected from the group consisting of -H, -CH₃, -NH₂, -OH, -F, β-D-ribofuranosyl, and 2-deoxy-β-D-ribofuranosyl. Alternatively, -R⁷ is -CH₃, and -R⁹ is no group; or -R⁷ is -CH₃, and -R⁹ is -X ¹. Particularly, they are each independently selected from the group consisting of -CH₃, -NH₂, -OH, -F, β-D-ribofuranosyl, and 2-deoxy-β-D-ribofuranosyl.

As used herein, "-̅ -̅ -̅ -̅ -̅ -̅" marked in the structural formula indicates a single bond or a double bond, including separate disclosures where it is a single bond and separate disclosures where it is a double bond.

In some embodiments, the first moiety is selected from a group consisting of formula (Ra), formula (Rb), formula (Ya), and formula (Yb),

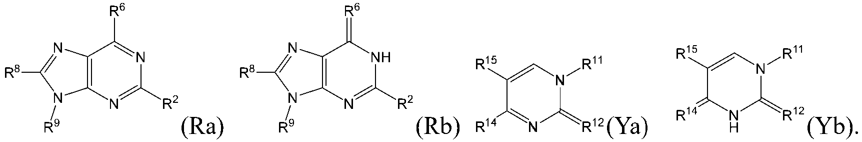

-R², -R⁸, -R⁹, -R¹¹, and -R¹⁵ are -X¹, and particularly, each independently selected from the group consisting of -H, -CH₃, -NH₂, -OH, -F, β-D-ribofuranosyl, and 2-deoxy-β-D-ribofuranosyl. =R¹² is =X², and particularly, selected from the group consisting of =CH₂, =NH, and =O. R⁶ is - X¹ in formula (Ra) and =X² in formula (Rb). Particularly, in formula (Ra), -R⁶ is selected from the group consisting of -H, -CH₃, -NH₂, -OH, -F, β-D-ribofuranosyl, and 2-deoxy-β-D-ribofuranosyl, and in formula (Rb), =R⁶ is selected from the group consisting of =CH₂, =NH, and =O. In addition, R¹⁴ is -X¹ in formula (Ya) and =X² in formula (Yb). Particularly, in formula (Ya), -R¹⁴ is selected from the group consisting of -H, -CH₃, -NH₂, -OH, -F, β-D-ribofuranosyl, and 2-deoxy-β-D-ribofuranosyl, and in formula (Yb), =R¹⁴ is selected from the group consisting of =CH₂, =NH, and =O.

In some embodiments, R¹ is no group.

In some embodiments, R² is selected from the group consisting of -H, -CH₃, -NH₂, -OH, - F, β-D-ribofuranosyl, and 2-deoxy-β-D-ribofuranosyl. In some embodiments, R² is -H or -NH₂. In some embodiments, R² is -H. In some embodiments, R² is -CH₃. In some embodiments, R² is - NH₂. In some embodiments, R² is -OH. In some embodiments, R² is -F. In some embodiments, R² is β-D-ribofuranosyl. In some embodiments, R² is 2-deoxy-β-D-ribofuranosyl.

In some embodiments, R³ is no group.

In some embodiments, R⁶ is selected from the group consisting of -H, -CH₃, =CH₂, -NH₂, =NH, -OH, =O, -F, β-D-ribofuranosyl, and 2-deoxy-β-D-ribofuranosyl. In some embodiments, R⁶ is selected from a group consisting of -H, -CH₃, -NH₂, -OH, -F, and β-D-ribofuranosyl. In some embodiments, R⁶ is selected from the group consisting of =CH₂, =NH, and =O. In some embodiments, R⁶ is -NH₂ or =O. In some embodiments, R⁶ is -H. In some embodiments, R⁶ is - CH₃. In some embodiments, R⁶ is -NH₂. In some embodiments, R⁶ is -OH. In some embodiments, R⁶ is -F. In some embodiments, R⁶ is β-D-ribofuranosyl. In some embodiments, R⁶ is 2-deoxy-β-D-ribofuranosyl. In some embodiments, R⁶ is =CH₂. In some embodiments, R⁶ is =NH. In some embodiments, R⁶ is =O.

In some embodiments, R⁷ is no group. In some embodiments, R⁷ is selected from the group consisting of -H, -CH₃, -NH₂, -OH, -F, β-D-ribofuranosyl, and 2-deoxy-β-D-ribofuranosyl. In some embodiments, R⁷ is selected from a group consisting of -CH₃, -NH₂, -OH, -F, β-D-ribofuranosyl, and 2-deoxy-β-D-ribofuranosyl. In some embodiments, R⁷ is -H or β-D-ribofuranosyl. In some embodiments, R⁷ is -H. In some embodiments, R⁷ is -CH₃. In some embodiments, R⁷ is -NH₂. In some embodiments, R⁷ is -OH. In some embodiments, R⁷ is -F. In some embodiments, R⁷ is β-D-ribofuranosyl. In some embodiments, R⁷ is 2-deoxy-β-D-ribofuranosyl.

In some embodiments, R⁸ is selected from the group consisting of -H, -CH₃, -NH₂, -OH, - F, β-D-ribofuranosyl, and 2-deoxy-β-D-ribofuranosyl. In some embodiments, R⁸ is -H. In some embodiments, R⁸ is -CH₃. In some embodiments, R⁸ is -NH₂. In some embodiments, R⁸ is -OH. In some embodiments, R⁸ is -F. In some embodiments, R⁸ is β-D-ribofuranosyl. In some embodiments, R⁸ is 2-deoxy-β-D-ribofuranosyl.

In some embodiments, R⁹ is no group. In some embodiments, R⁹ is selected from the group consisting of -H, -CH₃, -NH₂, -OH, -F, β-D-ribofuranosyl, and 2-deoxy-β-D-ribofuranosyl. In some embodiments, R⁹ is selected from the group consisting of -CH₃, -NH₂, -OH, -F, β-D-ribofuranosyl, and 2-deoxy-β-D-ribofuranosyl. In some embodiments, R⁹ is -H or β-D-ribofuranosyl. In some embodiments, R⁹ is -H. In some embodiments, R⁹ is -CH₃. In some embodiments, R⁹ is -NH₂. In some embodiments, R⁹ is -OH. In some embodiments, R⁹ is -F. In some embodiments, R⁹ is β-D-ribofuranosyl. In some embodiments, R⁹ is 2-deoxy-β-D-ribofuranosyl.

In some embodiments, R¹¹ is selected from the group consisting of -H, -CH₃, -NH₂, -OH, - F, β-D-ribofuranosyl, and 2-deoxy-β-D-ribofuranosyl. In some embodiments, R¹¹ is -H, -CH₃, or β-D-ribofuranosyl. In some embodiments, R¹¹ is -H or β-D-ribofuranosyl. In some embodiments, R¹¹ is -H. In some embodiments, R¹¹ is -CH₃. In some embodiments, R¹¹ is -NH₂. In some embodiments, R¹¹ is -OH. In some embodiments, R¹¹ is -F. In some embodiments, R¹¹ is β-D-ribofuranosyl. In some embodiments, R¹¹ is 2-deoxy-β-D-ribofuranosyl.

In some embodiments, R¹² is selected from the group consisting of =CH₂, =NH, and =O. In some embodiments, R¹² is =CH₂. In some embodiments, R¹² is =NH. In some embodiments, R¹² is =O.

In some embodiments, R¹³ is no group or -H. In some embodiments, R¹³ is no group. In some embodiments, R¹³ is -H.

In some embodiments, R¹⁴ is selected from the group consisting of -H, -CH₃, =CH₂, -NH₂, =NH, -OH, =O, -F, β-D-ribofuranosyl, and 2-deoxy-β-D-ribofuranosyl. In some embodiments, R¹⁴ is selected from the group consisting of -H, -CH₃, -NH₂, -OH, -F, and β-D-ribofuranosyl. In some embodiments, R¹⁴ is selected from the group consisting of =CH₂, =NH, and =O. In some embodiments, R¹⁴ is -NH₂ or =O. In some embodiments, R¹⁴ is -H. In some embodiments, R¹⁴ is -CH₃. In some embodiments, R¹⁴ is -NH₂. In some embodiments, R¹⁴ is -OH. In some embodiments, R¹⁴ is -F. In some embodiments, R¹⁴ is β-D-ribofuranosyl. In some embodiments, R¹⁴ is 2-deoxy-β-D-ribofuranosyl. In some embodiments, R¹⁴ is =CH₂. In some embodiments, R¹⁴ is =NH. In some embodiments, R¹⁴ is =O.

In some embodiments, R¹⁵ is selected from the group consisting of -H, -CH₃, -NH₂, -OH, - F, β-D-ribofuranosyl, and 2-deoxy-β-D-ribofuranosyl. In some embodiments, R¹⁵ is -H, -CH₃, or -F. In some embodiments, R¹⁵ is -H or -CH₃. In some embodiments, R¹⁵ is -H. In some embodiments, R¹⁵ is -CH₃. In some embodiments, R¹⁵ is -F.

In some embodiments, R¹⁶ is no group.

In some embodiments, the first moiety is selected from a group consisting of adenine, guanine, 7-methylguanine, hypoxanthine, xanthine, cytosine, 5-methylcytosine, uracil, thymine, 5-fluorouracil, 5,6-dihydrouracil, adenosine, guanosine, 7-methylguanosine, inosine, xanthosine, cytidine, 5-methylcytidine, uridine, thymidine, 5-fluorouridine, 5,6-dihydrouridine, deoxyadenosine, deoxyguanosine, 7-methyldeoxyguanosine, deoxyinosine, deoxyxanthosine, deoxycytidine, 5-methyldeoxycytidine, deoxyuridine, deoxythymidine, 5-fluorodeoxyuridine, and 5,6-dihydrodeoxyuridine. In some embodiments, the first moiety is selected from a group consisting of adenine, guanine, hypoxanthine, cytosine, uracil, thymine, 5-fluorouracil, adenosine, guanosine, inosine, cytidine, uridine, thymidine, and 5-fluorouridine. In some embodiments, the first moiety is selected from a group consisting of adenine, guanine, hypoxanthine, cytosine, uracil, thymine, adenosine, guanosine, inosine, cytidine, uridine, and thymidine.

In some embodiments, the Q¹ moiety is adenine. In some embodiments, the Q¹ moiety is guanine. In some embodiments, the Q¹ moiety is 7-methylguanine. In some embodiments, the Q¹ moiety is hypoxanthine. In some embodiments, the Q¹ moiety is xanthine. In some embodiments, the Q¹ moiety is cytosine. In some embodiments, the Q¹ moiety is 5-methylcytosine. In some embodiments, the Q¹ moiety is uracil. In some embodiments, the Q¹ moiety is thymine. In some embodiments, the Q¹ moiety is 5-fluorouracil. In some embodiments, the Q¹ moiety is 5,6-dihydrouracil. In some embodiments, the Q¹ moiety is adenosine. In some embodiments, the Q¹ moiety is guanosine. In some embodiments, the Q¹ moiety is 7-methylguanosine. In some embodiments, the Q¹ moiety is inosine. In some embodiments, the Q¹ moiety is xanthosine. In some embodiments, the Q¹ moiety is cytidine. In some embodiments, the Q¹ moiety is 5-methylcytidine. In some embodiments, the Q¹ moiety is uridine. In some embodiments, the Q¹ moiety is thymidine. In some embodiments, the Q¹ moiety is 5-fluorouridine. In some embodiments, the Q¹ moiety is 5,6-dihydrouridine. In some embodiments, the Q² moiety is adenine. In some embodiments, the Q² moiety is guanine. In some embodiments, the Q² moiety is 7-methylguanine. In some embodiments, the Q² moiety is hypoxanthine. In some embodiments, the Q² moiety is xanthine. In some embodiments, the Q² moiety is cytosine. In some embodiments, the Q² moiety is 5-methylcytosine. In some embodiments, the Q² moiety is uracil. In some embodiments, the Q² moiety is thymine. In some embodiments, the Q² moiety is 5-fluorouracil. In some embodiments, the Q² moiety is 5,6-dihydrouracil. In some embodiments, the Q² moiety is adenosine. In some embodiments, the Q² moiety is guanosine. In some embodiments, the Q² moiety is 7-methylguanosine. In some embodiments, the Q² moiety is inosine. In some embodiments, the Q² moiety is xanthosine. In some embodiments, the Q² moiety is cytidine. In some embodiments, the Q² moiety is 5-methylcytidine. In some embodiments, the Q² moiety is uridine. In some embodiments, the Q² moiety is thymidine. In some embodiments, the Q² moiety is 5-fluorouridine. In some embodiments, the Q² moiety is 5,6-dihydrouridine.

In some embodiments, the first moiety is selected from the group consisting of 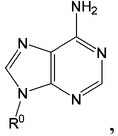 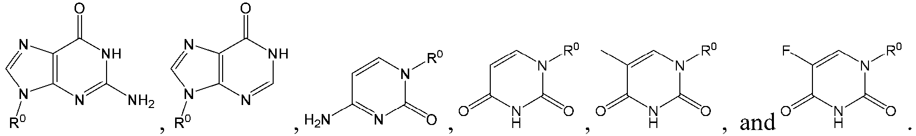 R⁰ is selected from the group consisting of -H, β-D-ribofuranosyl, and 2-deoxy-β-D-ribofuranosyl, and particularly, selected from the group consisting of -H and β-D-ribofuranosyl.

In some embodiments, the first moiety is selected from a group consisting of 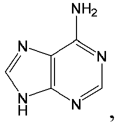 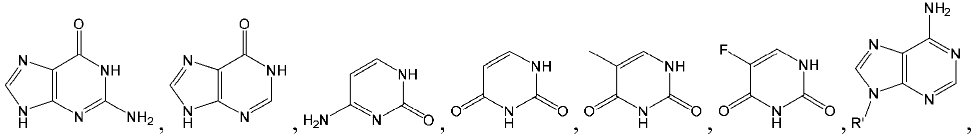 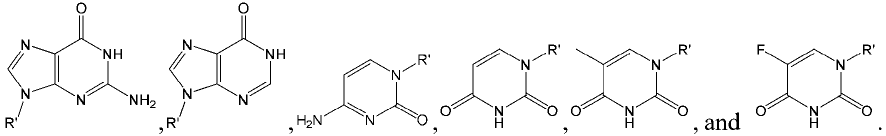 R' is β-D-ribofuranosyl.

In some embodiments, the ⁺Q¹-H moiety is selected from a group consisting of 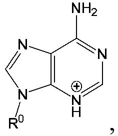 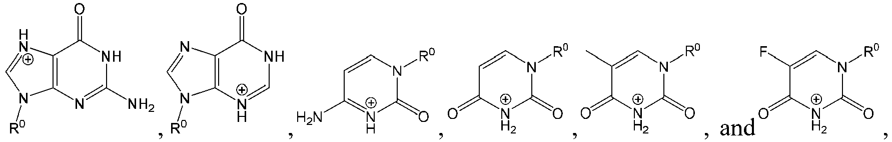 wherein R⁰ is selected from the group consisting of -H, β-D-ribofuranosyl, and 2-deoxy-β-D-ribofuranosyl, and particularly, selected from the group consisting of -H and β-D-ribofuranosyl. The N⁺ covalently attached hydrogen is the hydrogen in which the ⁺Q¹-H moiety is attached to the Q² moiety in formula [⁺Q¹-H···Q²], *i.e.,* "H" in "⁺Q¹-H".

In some embodiments, the Q² moiety is selected from the group consisting of 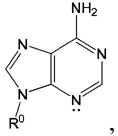 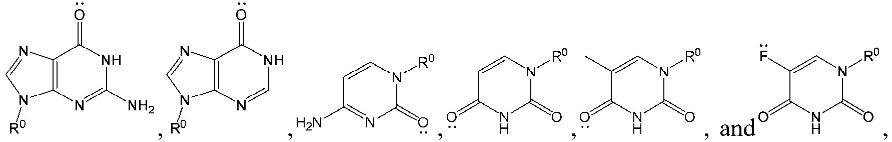 wherein R⁰ is selected from the group consisting of -H, β-D-ribofuranosyl, and 2-deoxy-β-D-ribofuranosyl, and particularly, selected from the group consisting of -H and β-D-ribofuranosyl. The atom marked with the lone pair of electrons is the atom in which the Q² moiety is attached to the ⁺Q¹-H moiety in formula [⁺Q¹-H···Q²].

In some embodiments, -L² is H, or is connected to -L¹ to form a ring.

In some embodiments, -L² is H, and -L¹ is selected from a group consisting of hydrogen, methyl, hydroxymethyl, mercaptomethyl, selenomethyl, aminomethyl, carboxymethyl, carbamoylmethyl, methylthiomethyl, guanidinomethyl, ethyl, 1-hydroxyethyl, 2-hydroxyethyl, 1-mercaptoethyl, 2-mercaptoethyl, 1-aminoethyl, 2-aminoethyl, 1-carboxyethyl, 2-carboxyethyl, 1-carbamoylethyl, 2-carbamoylethyl, 1-methylthioethyl, 2-methylthioethyl, 1-guanidinoethyl, 2-guanidinoethyl, n-propyl, isopropyl, 1-hydroxypropyl, 2-hydroxypropyl, 3-hydroxypropyl, 1-mercaptopropyl, 2-mercaptopropyl, 3-mercaptopropyl, 1-aminopropyl, 2-aminopropyl, 3-aminopropyl, 1-carboxypropyl, 2-carboxypropyl, 3-carboxypropyl, 1-carbamoylpropyl, 2-carbamoylpropyl, 3-carbamoylpropyl, 1-methylthiopropyl, 2-methylthiopropyl, 3-methylthiopropyl, 1-guanidinopropyl, 2-guanidinopropyl, 3-guanidinopropyl, n-butyl, isobutyl, sec-butyl, tert-butyl, 4-aminobutyl, 1-hydroxybutyl, 2-hydroxybutyl, 3-hydroxybutyl, 4-hydroxybutyl, 1-mercaptobutyl, 2-mercaptobutyl, 3-mercaptobutyl, 4-mercaptobutyl, 1-aminobutyl, 2-aminobutyl, 3-aminobutyl, 4-aminobutyl, 1-carboxybutyl, 2-carboxybutyl, 3-carboxybutyl, 4-carboxybutyl, 1-carbamoylbutyl, 2-carbamoylbutyl, 3-carbamoylbutyl, 4-carbamoylbutyl, 1-methylthiobutyl, 2-methylthiobutyl, 3-methylthiobutyl, 4-methylthiobutyl, 1-guanidinobutyl, 2-guanidinobutyl, 3-guanidinobutyl, 4-guanidinobutyl, benzyl, 2-hydroxybenzyl, 3-hydroxybenzyl, 4-hydroxybenzyl, (pyrrol-4-yl)methyl, (indol-3-yl)methyl, and (3-methyl-3,4-dihydropyrrole-2-carbonyl)aminobutyl; or -L¹ and -L² together form a group selected from a group consisting of propane-1,3-diyl, butane-1,4-diyl, and pentane-1,5-diyl.

In some embodiments, -L² is H, and -L¹ is selected from a group consisting of hydrogen, methyl, hydroxymethyl, mercaptomethyl, selenomethyl, carboxymethyl, carbamoylmethyl, 1-hydroxyethyl, carboxyethyl, carbamoylethyl, 2-methylthioethyl, isopropyl, 3-guanidinopropyl, isobutyl, sec-butyl, 4-aminobutyl, benzyl, 4-hydroxybenzyl, (pyrrol-4-yl)methyl, (indol-3-yl)methyl, and (3-methyl-3,4-dihydropyrrole-2-carbonyl)aminobutyl; or -L¹ and -L² together form propane-1,3-diyl.

In some embodiments, the second moiety is amino acid. In some embodiments, the second moiety is L-amino acid. In some embodiments, the second moiety is D-amino acid. In some embodiments, the second moiety is selected from a group consisting of glycine, alanine, serine, cysteine, selenocysteine, aspartic acid, asparagine, threonine, glutamic acid, glutamine, methionine, valine, arginine, leucine, isoleucine, lysine, tyrosine, phenylalanine, histidine, tryptophan, proline, and pyrrolysine. In some embodiments, the second moiety is selected from a group consisting of glycine, L-alanine, L-serine, L-cysteine, L-selenocysteine, L-aspartic acid, L-asparagine, L-threonine, L-glutamic acid, L-glutamine, L-methionine, L-valine, L-arginine, L-leucine, L-isoleucine, L-lysine, L-tyrosine, L-phenylalanine, L-histidine, L-tryptophan, L-proline, L-pyrrolysine, D-alanine, D-serine, D-cysteine, D-selenocysteine, D-aspartic acid, D-asparagine, D-threonine, D-glutamic acid, D-glutamine, D-methionine, D-valine, D-arginine, D-leucine, D-isoleucine, D-lysine, D-tyrosine, D-phenylalanine, D-histidine, D-tryptophan, D-proline, and D-pyrrolysine. In some embodiments, the second moiety is selected from a group consisting of glycine, L-alanine, L-serine, L-cysteine, L-selenocysteine, L-aspartic acid, L-asparagine, L-threonine, L-glutamic acid, L-glutamine, L-methionine, L-valine, L-arginine, L-leucine, L-isoleucine, L-lysine, L-tyrosine, L-phenylalanine, L-histidine, L-tryptophan, L-proline, and L-pyrrolysine. In some embodiments, the second moiety is selected from a group consisting of glycine, D-alanine, D-serine, D-cysteine, D-selenocysteine, D-aspartic acid, D-asparagine, D-threonine, D-glutamic acid, D-glutamine, D-methionine, D-valine, D-arginine, D-leucine, D-isoleucine, D-lysine, D-tyrosine, D-phenylalanine, D-histidine, D-tryptophan, D-proline, and D-pyrrolysine.

In some embodiments, the second moiety is selected from a group consisting of alanine, asparagine, histidine, proline, pyrrolysine, serine, tryptophan, and tyrosine. In some embodiments, the second moiety is selected from a group consisting of L-alanine, L-asparagine, L-histidine, L-proline, L-pyrrolysine, L-serine, L-tryptophan, and L-tyrosine. In some embodiments, the second moiety is selected from a group consisting of histidine, pyrrolysine, proline, tryptophan, arginine, and lysine. In some embodiments, the second moiety is selected from a group consisting of histidine, pyrrolysine, proline, and tryptophan. In some embodiments, the second moiety is selected from a group consisting of aspartic acid, asparagine, glutamic acid, and glutamine.

In some embodiments, the ⁺Q¹-H moiety is non-covalently bonded to the Q ² moiety through protonation of a nitrogen atom therein, particularly through protonation of a nitrogen atom in a heterocyclic ring of the ⁺Q¹-H moiety. In some embodiments, the ⁺Q¹-H moiety is selected from a group consisting of 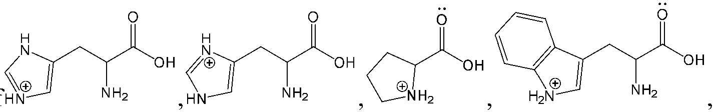 and 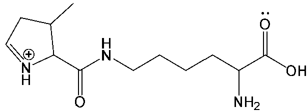 . The N⁺ covalently attached hydrogen is the hydrogen in which the ⁺Q¹-H moiety is attached to the Q² moiety in formula [⁺Q¹-H···Q²], *i.e.,* "H" in "⁺Q¹-H".

In some embodiments, the Q² moiety is non-covalently bonded to the ⁺Q¹-H moiety through =O: of the carbonyl, particularly, through =O: of the carboxyl. In some embodiments, the Q² moiety is selected from a group consisting of 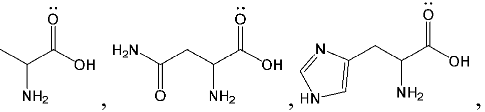 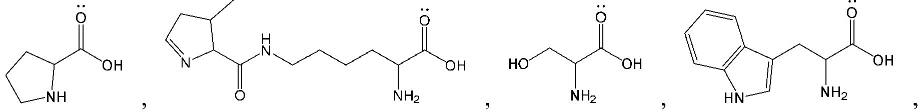 and 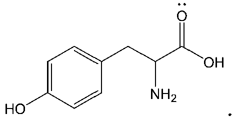 The atom marked with the lone pair of electrons is the atom in which the Q² moiety is attached to the ⁺Q¹-H moiety in formula [⁺Q¹-H···Q²].

Any disclosed or contained chemical structure specified in the present application shall be regarded as further disclosing or containing stereoisomers of the specified chemical structure, wherein any specific moieties Q¹ and Q² are selected from formula [⁺Q¹-H···Q²] and/or other structural formulae, substituents are R¹, R², R³, R⁶, R⁷, R⁸, and R⁹ when Q¹ is formula (R'), substituents are R¹¹, R¹², R¹³, R¹⁴, R¹⁵, and R¹⁶ when Q¹ is formula (Y'), or substituents are L and chiral when Q¹ is formula (A); and substituents are R¹, R², R³, R⁶, R⁷, R⁸, and R⁹ when Q² is formula (R'), substituents are R¹¹, R¹², R¹³, R¹⁴, R¹⁵, and R¹⁶ when Q² is formula (Y'), or substituents are L and chiral when Q² is formula (A). Any chemical structure disclosed or contained in the present application, especially in examples, shall be regarded as further disclosing or containing stereoisomers of the chemical structure.

In the present application, if the stereochemistry of any one or more chiral centers is not specified, it shall be regarded as disclosing or containing possible stereoisomers of the chiral center or combinations or mixtures of the stereoisomers. For example, if the stereochemistry of a chiral center is not specified, it shall be regarded as respectively disclosing or containing a chemical structure of a chiral center in an (*R*)-configuration, a chemical structure of a chiral center in an (*S*)-configuration, and a combination of the chemical structures in any ratio, including a racemic mixture. For example, if the stereochemistry of two chiral centers is not specified, it shall be regarded as respectively disclosing or containing a chemical structure of the two chiral centers in an (*R,R*)-configuration, a chemical structure of the two chiral centers in an (*S,S*)-configuration, a chemical structure of the two chiral centers in an (*R,S*)-configuration, a chemical structure of the two chiral centers in an (*S,R*)-configuration, and a combination of the chemical structures in any ratio.

As a specific example of the foregoing definition, any disclosed or contained chemical structure "including a moiety with a double bond or carbon on an aromatic ring and hydroxyl or sulfhydryl on carbon" shall be regarded as simultaneously and further disclosing or containing a corresponding tautomer, that is, a chemical structure including a "carbonyl or thiocarbonyl" moiety. For example, any disclosed or contained chemical structure including a " 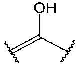 " moiety shall be regarded as further disclosing or containing a corresponding tautomer including a " 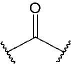 " moiety, and any disclosed or contained chemical structure including a " 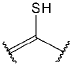 " moiety shall be regarded as further disclosing or containing a corresponding tautomer including a " 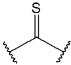 " moiety. Any disclosed or contained chemical structure including an enol moiety of nucleobase shall be regarded as further disclosing or containing a corresponding chemical structure including a keto moiety of nucleobase. Any disclosed or contained chemical structure including the keto moiety of nucleobase shall be regarded as further disclosing or containing a corresponding chemical structure including the enol moiety of nucleobase.

As a specific example of the foregoing definition, any disclosed or contained chemical structure including a " 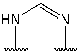 " moiety in an aromatic ring shall be regarded as simultaneously and further disclosing or containing a corresponding tautomer including a " 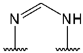 " moiety. For example, any disclosed or contained chemical structure including a " 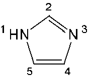 " moiety in an imidazole ring shall be regarded as further disclosing or containing a corresponding tautomer including a " 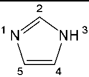 " moiety.

Any resonance structure constituting a hybrid that is disclosed or contained in the present application shall be regarded as further disclosing or containing all other resonance structures constituting the hybrid and the hybrid itself.

As a specific example of the foregoing definition, any disclosed or contained chemical structure including a " 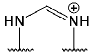 " moiety in an aromatic ring shall be regarded as simultaneously and further disclosing or containing a corresponding resonance chemical structure including a " 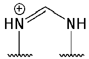 " moiety. For example, any disclosed or contained chemical structure including a " 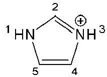 " moiety in the imidazole ring shall be regarded as further disclosing or containing a resonance chemical structure including a " 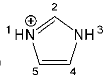 " moiety, and more further disclosing or containing a corresponding hybrid (for example, represented as " 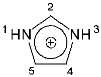 ").

Any chemical structure that is disclosed or contained in the present application and that includes a non-covalent bond, such as a hydrogen bond, that is formed by hydrogen on *N*¹ in " 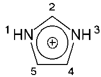 " in a protonated imidazole ring shall be regarded as simultaneously and further disclosing or containing a corresponding chemical structure including a non-covalent bond, such as a hydrogen bond, that is formed by hydrogen on *N*³ in " 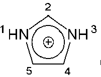 " in a protonated imidazole ring. Any chemical structure that is disclosed or contained in the present application and that includes a non-covalent bond, such as a hydrogen bond, that is formed by hydrogen on *N*^{τ} in protonated histidine, protonated histamine, protonated sarcosine, or a similar compound with 1*H-*imidazol-4-yl or a group derived from 1*H*-imidazol-4-yl shall be regarded as simultaneously and further disclosing or containing a corresponding chemical structure including a non-covalent bond, such as a hydrogen bond, that is formed by hydrogen on *N*^{τ} in protonated histidine, protonated histamine, protonated sarcosine, or a similar compound with 1*H*-imidazol-4-yl or a group derived from 1*H*-imidazol-4-yl.

Any (Bronsted-Lowry) conjugate acid or base disclosed or contained in the present application shall be regarded as further disclosing or containing its corresponding or dominant conjugate acid-base pair under certain pH conditions in a case of being chemically reasonable and not affecting the main chemical structure and technical effects of the non-covalent dimer cation or salt of the present application. For example, in a case of being chemically reasonable and not affecting the main chemical structure and technical effects of the non-covalent dimer cation or salt of the present application, any disclosed or contained specific moiety or group that is hydroxyl shall be regarded as further disclosing or containing a specific moiety or group that is an oxygen ion group (-O⁻) or oxygen (-OH₂⁺). In a case of being chemically reasonable and not affecting the main chemical structure and technical effects of the non-covalent dimer cation or salt of the present application, any disclosed or contained specific moiety or group that is an amino group shall be regarded as further disclosing or containing a specific moiety or group that is an amino anion group (-NH⁻) or ammonium (-NH₃⁺).

### Salt of the present application

In an aspect, the present application provides a salt.

In some embodiments, the salt of the present application comprises the non-covalent dimer cation of the present application, and particularly, comprises the non-covalent dimer cation of the present application and a first anion. For example, the salt of the present application may comprise or consist of formula [⁺Q¹-H···Q²]*ₖ*X, wherein X is the first anion.

Through the first anion of the present application, the cation of the present application is enabled to exist stably in the form of a salt.

In some embodiments, the first anion (X in formula [⁺Q¹-H···Q²]*ₖ*X) comprises or consists of at least one selected from a group consisting of a fluoride ion, a chloride ion, a bromide ion, an iodide ion, a sulfide ion, a nitrate ion, a sulfate ion, a sulfite ion, a thiosulfate ion, a persulfate ion, a selenate ion, a phosphate ion, a carbonate ion, a hexafluorophosphate ion, a hexafluorosilicate ion, an acetate ion, a succinate ion, a sulfonate ion, a benzoate ion, and a polyphosphate ion. In some embodiments, the first anion comprises or consists of at least one selected from a group consisting of a hexafluorophosphate ion, an acetate ion, a succinate ion, a sulfate ion, a phosphate ion, and a chloride ion. In some embodiments, the first anion is a fluoride ion. In some embodiments, the first anion is a chloride ion. In some embodiments, the first anion is a bromide ion. In some embodiments, the first anion is an iodide ion. In some embodiments, the first anion is a sulfide ion. In some embodiments, the first anion is a nitrate ion. In some embodiments, the first anion is a sulfate ion. In some embodiments, the first anion is a sulfite ion. In some embodiments, the first anion is a thiosulfate ion. In some embodiments, the first anion is a persulfate ion. In some embodiments, the first anion is a selenate ion. In some embodiments, the first anion is a phosphate ion. In some embodiments, the first anion is a carbonate ion. In some embodiments, the first anion is a hexafluorophosphate ion. In some embodiments, the first anion is a hexafluorosilicate ion. In some embodiments, the first anion is an acetate ion. In some embodiments, the first anion is a succinate ion. In some embodiments, the first anion is a sulfonate ion, such as a sulfamate ion. In some embodiments, the first anion is a benzoate ion. In some embodiments, the first anion is a polyphosphate ion.

A value of a parameter *k* is obtained based on the valence of the non-covalent dimer cation of the present application and the valence of the first anion. In a case of the non-covalent dimer cation of the present application with a monovalent positive electron, the value of the parameter *k* is equal to the valence of the first anion. In some embodiments, *k* is 1. In some embodiments, *k* is 2. In some embodiments, *k* is 3.

### Method of preparing the non-covalent dimer cation of the present application or the salt of the present application

In some embodiments, a method of preparing a salt comprising the non-covalent dimer cation of the present application, *i.e.,* the salt of the present application, is provided, which may comprise the following steps.

### Salt formation of first reactant

A first reactant is contacted with a first acid. Then, a first acid salt of the first reactant is obtained. In some embodiments, the first acid salt of the first reactant comprises or consists of the first reactant and the first acid, and/or a conjugate base (a conjugate base of the first acid) of a protonated first reactant and the first acid.

In some embodiments, the first reactant comprises or essentially consists of the compound Q¹, and particularly, consists of the compound Q¹. In some embodiments, the first acid salt of the first reactant comprises or essentially consists of a salt Q¹ · *p*H*ₐ*A, and particularly, consists of the salt Q¹ · *p*H*ₐ*A, wherein H*ₐ*A is the first acid, A is the conjugate base of the first acid, *a* is the valence of the conjugate base of the first acid, and *p* is the number of equivalents of the first acid H*ₐ*A. R¹, R², R³, R⁶, R⁷, R⁸, R⁹, R¹¹, R¹², R¹³, R¹⁴, R¹⁵, R¹⁶, and L (if any) in the compound Q¹ or salt Q¹ · *p*H*ₐ*A may be consistent with corresponding moieties or parameters of a desired cation of formula [⁺Q¹-H···Q²] or salt of formula [⁺Q¹-H···Q²]*ₖ*X according to examples of the present application.

In some embodiments, the first reactant is contacted with the first acid in the presence of a first solvent, and particularly, in the first solvent. In some embodiments, the first acid is at least one selected from a group consisting of nitric acid, phosphoric acid, sulfuric acid, hydrofluoric acid, hydrochloric acid, hydrobromic acid, and hydroiodic acid, especially hydrochloric acid. That is, the conjugate base A of the first acid is at least one selected from a group consisting of a nitrate ion, a phosphate ion, a hydrogen phosphate ion, a dihydrogen phosphate ion, a sulfate ion, a hydrogen sulfate ion, a fluoride ion, a chloride ion, a bromide ion, and an iodide ion, especially a chloride ion. In some embodiments, the first solvent is water. The parameters *p* and *a* in the salt Q¹ · *p*H*ₐ*A are obtained based on the type of the first acid. For example, in some embodiments, the first acid is hydrochloric acid, and *p* is 1.

As used herein, unless otherwise further defined, the term "conjugate acid" is intended to comprise the Bronsted-Lowry conjugate acid of the compound to which the term is directed. Furthermore, it does not only comprise a first conjugate acid obtained in a case that the compound acquires a proton, but also comprise, if chemically allowed, an acid obtained in a case that the compound further acquires more protons, such as a second conjugate acid obtained in a case that the first conjugate further acquires a proton, and a third conjugate acid obtained in a case that the second conjugate acid much further acquires a proton, and so on. Unless otherwise further defined, the term "conjugate base" herein is intended to comprise the Bronsted-Lowry conjugate base of the compound to which the term is directed. Further, it does not only comprise a first conjugate base obtained in a case that the compound loses a proton, but also comprises, if chemically allowed, a base obtained in a case that the compound further loses more protons, such as a second conjugate base obtained in a case that the first conjugate base further loses a proton, and a third conjugate base obtained in a case that the second conjugate base much further loses a proton, and so on. For example, unless otherwise further defined, a conjugate base of sulfuric acid herein shall be regarded as comprising not only a hydrogen sulfate ion, but also a sulfate ion. For example, unless otherwise further defined, a conjugate base of phosphoric acid herein shall be regarded as comprising not only a hydrogen phosphate ion, but also a dihydrogen phosphate ion and a phosphate ion.

In some embodiments, the first acid is in excess relative to the first reactant. In some embodiments, a molar ratio of the first reactant to the first acid is 1: 2 to 1: 10, particularly 1: 2.5 to 1: 8, more particularly 1: 4 to 1: 6, and more particularly about 1: 5.

In some embodiments, the concentration of the first acid in the first solvent is more than 1 M. In some embodiments, the concentration of the first acid in the first solvent is 1 M to 5 M. In some embodiments, the concentration of the first acid in the first solvent is about 2 M.

In some embodiments, the first reactant is contacted with the first acid at a first temperature. In some embodiments, the first temperature is 0°C to 70°C. In some embodiments, the first temperature is 10°C to 40°C, more particularly 20°C to 30°C, and more particularly about 25°C. In some embodiments, the first temperature is 20°C to 50°C, more particularly 30°C to 40°C, and more particularly about 37°C. In some embodiments, the first temperature is 0°C to 20°C, more particularly 0°C to 10°C, and more particularly about 4°C. In some embodiments, the first temperature is 40°C to 70°C, more particularly 50°C to 60°C, and more particularly about 60°C.

In some embodiments, after the first reactant is contacted with the first acid, heating and recrystallization are performed to obtain the first acid salt of the first reactant. In some embodiments, the heating temperature is 70°C to 100°C, particularly 80°C to 90°C, and more particularly about 85°C.

### Protonation of first acid salt of first reactant

In a first acidic environment, the first acid salt of the first reactant is contacted with a first salt that comprises a first anion, and particularly, consists of a first cation and the first anion. Then, a first solution that comprises a protonated first acid salt cation of the first reactant and the first anion of formula [⁺Q¹-H · *p*H*ₐ*A]*ₖ*X is obtained.

X and *k* in formula [⁺Q¹-H · *p*H*ₐ*A]*ₖ*X may be consistent with corresponding moieties or parameters of a desired salt of formula [⁺Q¹-H···Q²]*ₖ*X according to examples of the present application.

In some embodiments, the first salt comprises or consists of at least one selected from a group consisting of ammonium fluoride, ammonium chloride, ammonium bromide, ammonium iodide, ammonium sulfide, ammonium nitrate, ammonium sulfate, ammonium sulfite, ammonium thiosulfate, ammonium persulfate, ammonium selenate, ammonium phosphate, ammonium carbonate, ammonium hexafluorophosphate, ammonium hexafluorosilicate, ammonium acetate, any ammonium sulfonate, ammonium benzoate, and ammonium polyphosphate. In some embodiments, the first salt comprises or consists of at least one selected from a group consisting of ammonium hexafluorophosphate, ammonium hexafluorosilicate, ammonium chloride, and ammonium sulfate. In some embodiments, the first salt is ammonium fluoride. In some embodiments, the first salt is ammonium chloride. In some embodiments, the first salt is ammonium bromide. In some embodiments, the first salt is ammonium iodide. In some embodiments, the first salt is ammonium sulfide. In some embodiments, the first salt is ammonium nitrate. In some embodiments, the first salt is ammonium sulfate. In some embodiments, the first salt is ammonium sulfite. In some embodiments, the first salt is ammonium thiosulfate. In some embodiments, the first salt is ammonium persulfate. In some embodiments, the first salt is ammonium selenate. In some embodiments, the first salt is ammonium phosphate. In some embodiments, the first salt is ammonium carbonate. In some embodiments, the first salt is ammonium hexafluorophosphate. In some embodiments, the first salt is ammonium hexafluorosilicate. In some embodiments, the first salt is ammonium acetate. In some embodiments, the first salt is any ammonium sulfonate, such as ammonium sulfamate. In some embodiments, the first salt is ammonium benzoate. In some embodiments, the first salt is ammonium polyphosphate.

In some embodiments, the first acid salt of the first reactant is contacted with the first salt in the presence of a second solvent, and particularly, in the second solvent. In some embodiments, the second solvent is water. In some embodiments, the first cation is an ammonium ion. Here, the ammonium ion, as the first cation, may be taken as a hydrogen donor.

In some embodiments, the first salt is in excess relative to the first acid salt of the first reactant. In some embodiments, a molar ratio of the first acid salt of the first reactant to the first salt is 1: 2 to 1: 10, particularly 1: 2.5 to 1: 8, more particularly 1: 4 to 1: 6, and more particularly about 1: 5.

In some embodiments, the first solution further comprises a conjugate base of the first acid. In some embodiments, the first solution further comprises the first cation. In some embodiments, the first solution further comprises the second solvent. In some embodiments, the first solution is essentially, and particularly, consisting of the protonated first acid salt cation of the first reactant, the first cation, the first anion, the conjugate base of the first acid, and the second solvent.

In some embodiments, the first acid salt of the first reactant is contacted with the first salt at a second temperature. In some embodiments, the second temperature is 0°C to 70°C. In some embodiments, the second temperature is 10°C to 40°C, more particularly 20°C to 30°C, and more particularly about 25°C. In some embodiments, the second temperature is 20°C to 50°C, more particularly 30°C to 40°C, and more particularly about 37°C. In some embodiments, the second temperature is 0°C to 20°C, more particularly 0°C to 10°C, and more particularly about 4°C. In some embodiments, the second temperature is 40°C to 70°C, more particularly 50°C to 60°C, and more particularly about 60°C. In some embodiments, the first temperature and the second temperature are equal.

In some embodiments, the first acidic environment has a pH of less than or equal to 6. In some embodiments, the first acidic environment has a pH of 4 to 6, particularly 4.5 to 5.5, particularly 4.8 to 5.2, particularly 4.9 to 5.1, and more particularly about 5. Generally, for the first anion that is less acidic or more alkaline, a first acidic environment with a lower pH is selected. In some embodiments, the first anion is a hexafluorophosphate ion, and the first acidic environment has a pH of 4 to 6, particularly 4.5 to 5.5, particularly 4.8 to 5.2, particularly 4.9 to 5.1, more particularly about 5.

In some embodiments, the first acidic environment is obtained by adding the first salt to the second solvent. In some embodiments, the first acidic environment is obtained by adding the first salt and an acid consisting of the first anion and the hydrogen ion to the second solvent.

### Formation of non-covalent bond

In a second acidic environment, a second reactant is added to the first solution, and particularly, is contacted with a protonated first reactant cation in the first solution. Then, a bond is formed, and a second solution that comprises the cation (the first acid salt cation) according to the examples of the present application and the first anion of formula [⁺Q¹-H···Q² · *p*H*ₐ*A]*ₖ*X, is obtained.

In some embodiments, the second reactant comprises or essentially consists of the compound Q², and particularly, consists of the compound Q². R¹, R², R³, R⁶, R⁷, R⁸, R⁹, R¹¹, R¹², R¹³, R¹⁴, R¹⁵, and R¹⁶ (if any) may be consistent with corresponding moieties or parameters of a desired cation of formula [⁺Q¹-H···Q²] or salt of formula [⁺Q¹-H···Q²]*ₖ*X according to the examples of the present application.

In some embodiments, the second reactant is in excess relative to the protonated first reactant cation. In some embodiments, a molar ratio of the protonated first reactant cation to the second reactant is 1: 1 to 1: 2, such as about 1: 1.0, about 1: 1.1, about 1: 1.2, about 1: 1.3, about 1: 1.4, about 1: 1.5, about 1: 1.6, about 1: 1.7, about 1: 1.8, about 1: 1.9, or about 1: 2.0.

In some embodiments, the first solution further comprises a conjugate base of the first acid. In some embodiments, the first solution further comprises the first cation. In some embodiments, the first solution further comprises the second solvent. In some embodiments, the first solution is essentially, and particularly, consisting of the protonated first acid salt cation of the first reactant, the first cation, the first anion, the conjugate base of the first acid, and the second solvent.

In some embodiments, the second reactant is added to the first solution at a third temperature. In some embodiments, the third temperature is 0°C to 70°C. In some embodiments, the third temperature is 10°C to 40°C, more particularly 20°C to 30°C, and more particularly about 25°C. In some embodiments, the third temperature is 20°C to 50°C, more particularly 30°C to 40°C, and more particularly about 37°C. In some embodiments, the third temperature is 0°C to 20°C, more particularly 0°C to 10°C, and more particularly about 4°C. In some embodiments, the third temperature is 40°C to 70°C, more particularly 50°C to 60°C, and more particularly about 60°C. In some embodiments, the first temperature and the third temperature are equal. In some embodiments, the second temperature and the third temperature are equal. In some embodiments, the first temperature, the second temperature, and the third temperature are equal.

In some embodiments, the second acidic environment has a pH of less than or equal to 6. In some embodiments, the second acidic environment has a pH of 4 to 6, particularly 4.5 to 5.5, particularly 4.8 to 5.2, particularly 4.9 to 5.1, and more particularly about 5. Generally, for the first anion that is less acidic or more alkaline, a second acidic environment with a lower pH is selected. In some embodiments, the first anion is a hexafluorophosphate ion, and the second acidic environment has a pH of 4 to 6, particularly 4.5 to 5.5, particularly 4.8 to 5.2, particularly 4.9 to 5.1, more particularly about 5. In some embodiments, the first acidic environment and the second acidic environment have the same pH.

In some embodiments, the second acidic environment is obtained by adding the first salt to the second solvent. In some embodiments, the second acidic environment is obtained by adding the first salt and an acid consisting of the first anion and the hydrogen ion to the second solvent. In some embodiments, a reductant is further added to the first solution, and particularly, the reductant is added to the first solution before, after, or at the same time as the second reactant is added to the first solution. That is, in some embodiments, the second acidic environment is reductive, and particularly strongly reductive. The non-covalent dimer cation of the present application has a strong reducing capacity. Therefore, the addition of the reductant helps reduce a proportion of the non-covalent dimer cation of the present application that is oxidized during reaction, which is conducive to improving the yield of the salt of the present application. In some embodiments, the reductant comprises at least one or more selected from a group consisting of vitamin C, vitamin E, and derivatives thereof.

In some embodiments, the second solution is further purified to obtain a first intermediate comprising the cation according to the examples of the present application, *i.e.,* the first acid salt cation, and the first anion. In some embodiments, the purification comprises dialysis. In some embodiments, the dialysis may be performed through a membrane with MWCO of 50 Da to 500 Da, particularly about 100 Da or 200 Da.

### Formation of the salt of the present application

A first base is added to the second solution or contacted with the first intermediate to remove the first acid H*ₐ*A in the second solution or the first intermediate. Then, the salt of the present application is obtained.

In some embodiments, the salt of the present application comprises the non-covalent dimer cation of the present application and the first anion. For example, the salt of the present application may be of formula [⁺Q¹-H···Q²]*ₖ*X.

In some embodiments, the first base comprises or consists of, but is not limited to, at least one selected from a group consisting of ammonia water, sodium hydroxide, potassium hydroxide, and triethanolamine. A person skilled in the art should understand that any base that can adjust the pH and make a solution alkaline without causing a further redox reaction can be taken as the first base. In some embodiments, the first base is ammonia water, and particularly, dilute ammonia water. In some embodiments, the first base is sodium hydroxide. In some embodiments, the first base is potassium hydroxide. In some embodiments, the first base is triethanolamine.

In some embodiments, the adding the first base to the second solution or contacting the first base with the first intermediate refers to adjusting the pH of the second solution or a solution comprising the first intermediate to be alkaline. In some embodiments, the alkalinity refers to the pH being more than 8, particularly 8 to 10, particularly 8.5 to 9.5, particularly 8.8 to 9.2, and more particularly about 9.

In some embodiments, the contacting the first intermediate with the first base refers to contacting the first intermediate with the first base in water.

In some embodiments, after the first base is added to the second solution, purification is further performed to obtain the salt of the present application. In some embodiments, the purification comprises dialysis. In some embodiments, the dialysis may be performed through a membrane with MWCO of 50 Da to 500 Da, particularly about 100 Da or 200 Da. In some embodiments, the purification comprises filtration.

### Use of the non-covalent dimer cation of the present application or the salt of the present application

In some embodiments, use of the cation or salt of the present application in promotion of cell repair is provided. In some embodiments, use of the cation or salt of the present application in preparation of a medicament for promoting cell repair is provided. In some embodiments, a method for promoting cell repair is provided, comprising administrating the cation or salt of the present application. In some embodiments, the cation or salt of the present application is provided for promoting cell repair.

In some embodiments, antioxidation use of the cation or salt of the present application is provided. In some embodiments, use of the cation or salt of the present application in preparation of an antioxidant is provided. In some embodiments, an antioxidation method is provided, comprising administrating the cation or salt of the present application. In some embodiments, the cation or salt of the present application is provided for antioxidation. In some embodiments, the antioxidant is a medicament for inhibiting, reducing, or reversing oxidative stress in human or animal cells. In some embodiments, the antioxidant is a medicament for inhibiting, reducing, or scavenging oxygen species in human or animal bodies.

In some embodiments, use of the cation or salt of the present application in inhibiting, reducing, or scavenging oxygen species in human or animal bodies is provided. In some embodiments, use of the cation or salt of the present application in preparation of a medicament for inhibiting, reducing, or scavenging oxygen species in human or animal bodies is provided. In some embodiments, a method for inhibiting, reducing, or scavenging oxygen species in human or animal bodies is provided, comprising administrating the cation or salt of the present application, particularly to the human or animal cells. In some embodiments, the cation or salt of the present application is provided for inhibiting, reducing, or scavenging oxygen species in human or animal bodies.

In some embodiments, use of the cation or salt of the present application in treating an oxygen species-associated disease or symptom is provided. In some embodiments, use of the cation or salt of the present application in preparation of a medicament for treating an oxygen species-associated disease or symptom is provided. In some embodiments, a method for treating an oxygen species-associated disease or symptom is provided, comprising administrating the cation or salt of the present application, particularly to a patient with the oxygen species-associated disease or symptom. In some embodiments, the cation or salt of the present application is provided for treating the oxygen species-associated disease or symptom.

In some embodiments, the promotion of cell repair comprises, but is not limited to: the treatment of ageing, inflammation, overweight, obesity, cancer, tumor, hepatopathy, neurodegenerative disease (e.g., Parkinson's disease or Alzheimer's disease), arterial hypertension, atherosclerosis, cardiovascular disease, diabetes, hypercholesterolemia, chronic fatigue syndrome, ischemia-reperfusion damage, ultraviolet-induced damage, or alcohol-induced damage. In some embodiments, the promotion of cell repair is the treatment of mucosal damage, particularly gastric mucosal damage. In some embodiments, the promotion of cell repair is the treatment of alcohol-induced damage, particularly alcohol-induced gastric mucosal damage.

In some embodiments, the oxygen species-associated disease or symptom comprises, but is not limited to, ageing, inflammation, overweight, obesity, cancer, tumor, hepatopathy, neurodegenerative disease (e.g., Parkinson's disease or Alzheimer's disease), arterial hypertension, atherosclerosis, cardiovascular disease, diabetes, hypercholesterolemia, chronic fatigue syndrome, ischemia-reperfusion damage, ultraviolet-induced damage, or alcohol-induced damage. In some embodiments, the oxygen species-associated disease or symptom is gastric mucosal damage. In some embodiments, the oxygen species-associated disease or symptom is alcohol-induced damage, particularly, alcohol-induced gastric mucosal damage.

In some embodiments, the oxygen species-associated disease or symptom is neurodegenerative disease. In some embodiments, the neurodegenerative disease is Parkinson's disease or Alzheimer's disease. In some embodiments, the neurodegenerative disease is Parkinson's disease. In some embodiments, the neurodegenerative disease is Alzheimer's disease. In some embodiments, the neurodegenerative disease comprises a disease associated with increase of 2-phenyl-4,5-dihydro-4,4,5,5-tetramethyl-1*H*-imidazolyl-1-oxyl-3-oxide, or increase of 6-hydroxydopamine, and/or increase of β amyloid protein. In some embodiments, the neurodegenerative disease comprises a disease associated with increase of 2-phenyl-4,5-dihydro-4,4,5,5-tetramethyl-1*H*-imidazolyl-1-oxyl-3-oxide. In some embodiments, the neurodegenerative disease comprises a disease associated with increase of 6-hydroxydopamine. In some embodiments, the neurodegenerative disease comprises a disease associated with increase of β amyloid protein.

In some embodiments, the cation or salt of the present application may effectively act on a repair-promoting signaling pathway in a cell, enhance the cell self-repair function, and make the cell have the self-repair ability, thereby preventing, treating, or alleviating diseases that are susceptible to, such as Parkinson's disease, Alzheimer's disease, and other neurodegenerative diseases, due to the weakening of the cell repair ability in terms of cell functions.

In some embodiments, use of the cation or salt of the present application in promoting cell proliferation is provided. In some embodiments, use of the cation or salt of the present application in preparation of a medicament for promoting cell proliferation is provided. In some embodiments, a method for promoting cell proliferation is provided, comprising administrating the cation or salt of the present application. In some embodiments, the cation or salt of the present application is provided for promoting cell proliferation. In some embodiments, the cells are cells located in the skin. In some embodiments, the cells are skin cells. In some embodiments, the cells are selected from a group consisting of epidermal fibroblasts, keratinocytes, vascular endothelial cells, and nerve cells. In some embodiments, the cells are epidermal fibroblasts. In some embodiments, the cells are keratinocytes. In some embodiments, the cells are vascular endothelial cells. In some embodiments, the cells are nerve cells, particularly nerve cells of the skin. In some embodiments, the cells belong to epithelial tissues. In some embodiments, the cells belong to connective tissues. In some embodiments, the cells belong to muscular tissues. In some embodiments, the cells belong to nerve tissues.

In some embodiments, use of the cation or salt of the present application in treating, alleviating, or repairing wounds is provided. In some embodiments, use of the cation or salt of the present application in preparation of a medicament for treating, alleviating, or repairing wounds is provided. In some embodiments, a method for treating, alleviating, or repairing wounds is provided, comprising administrating the cation or salt of the present application. In some embodiments, the cation or salt of the present application is provided for treating, alleviating, or repairing wounds. In some embodiments, the wounds comprise a skin wound.

In some embodiments, the cation or salt of the present application may activate signaling pathways that promote cell proliferation in cells of living organisms so that a variety of cells may accelerate cell proliferation activities under the premise of regulation, thereby promoting the healing and repair of wounds and improving the wound repair effect. In some embodiments, the cation or salt of the present application may improve and treat diseases associated with insufficient cell proliferation, or accelerate a wound healing process.

### EXAMPLES

In the following examples, if a compound of an example comprises a first reactant, a second reactant, a cation of this example, or a salt of this example and has chirality that is not explicitly specified, the compound may be a chiral mixture, such as a racemic compound. The structural formulae of the following examples are listed together after the examples.

### Example 1

A salt of example 1 is specifically shown in formula (1). That is, Q¹ is adenine, Q² is alanine, *k =* 1, and X is a hexafluorophosphate ion.

### Preparation method

The method of preparing the salt of this example is as follows, wherein:
- the first reactant was adenine;
- the second reactant was alanine; and
- the first salt was ammonium hexafluorophosphate, that is, a first anion was a hexafluorophosphate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 4 g of first salt of this example was added and stirred at 25°C for 6 h to obtain a first aqueous solution including a protonated hydrochloride cation of the first reactant and the first anion.

0.89 g of second reactant was put into the first aqueous solution at 25°C and stirred gently for 24 h to obtain a second aqueous solution including the cation of this example. The second aqueous solution was completely dialyzed with a dialysis bag with MWCO of 200 Da, and then dried to obtain a first intermediate. The first intermediate was dissolved in 10 mL of ultrapure water, dilute ammonia water was added to adjust the pH to 9, and a large amount of beige precipitates were precipitated, followed by filtration. The filter cake was washed twice with water and dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 1.6 g, and the productivity rate was about 85%.

The salt of this example was subjected to high-resolution mass spectrometry detection to obtain the cation of this example, with m/z = 225.11 for (C₈H₁₃O₂N₆)⁺.

### Example 2

A salt of example 2 is specifically shown in formula (2). That is, Q¹ is adenine, Q² is asparagine, *k* = 1, and X is a hexafluorophosphate ion.

### Preparation method

The method of preparing the salt of this example is as follows, wherein:
- the first reactant was adenine;
- the second reactant was asparagine; and
- the first salt was ammonium hexafluorophosphate, that is, a first anion was a hexafluorophosphate ion.

1 g of first reactant was placed into 10 mL of 2 M hydrochloric acid at 37°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 4 g of first salt of this example was added and stirred at 37°C for 12 h to obtain a first aqueous solution including a protonated hydrochloride cation of the first reactant and the first anion.

0.89 g of second reactant was put into the first aqueous solution at 37°C and stirred gently for 36 h to obtain a second aqueous solution including the cation of this example. A 1 M sodium hydroxide solution was added to the second aqueous solution to adjust the pH to 9. The second aqueous solution was completely dialyzed with a dialysis bag with MWCO of 200 Da. The dialyzed second aqueous solution was dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 1.2 g, and the productivity rate was about 63%.

The salt of this example was subjected to high-resolution mass spectrometry detection to obtain the cation of this example, with m/z = 268.12 for (C₉H₁₄O₃N₇)⁺.

### Example 3

A salt of example 3 is specifically shown in formula (3). That is, Q¹ is adenine, Q² is histidine, *k =* 1, and X is a hexafluorophosphate ion.

### Preparation method

The method of preparing the salt of this example is as follows, wherein:
- the first reactant was adenine;
- the second reactant was histidine; and
- the first salt was ammonium hexafluorophosphate, that is, a first anion was a hexafluorophosphate ion.

1 g of first reactant was placed into 10 mL of 2 M hydrochloric acid at 60°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 2 g of first salt of this example was added and stirred at 60°C for 6 h to obtain a first aqueous solution including a protonated hydrochloride cation of the first reactant and the first anion.

0.65 g of second reactant was put into the first aqueous solution at 60°C and stirred gently for 48 h to obtain a second aqueous solution including the cation of this example. A 1 M sodium hydroxide solution was added to the second aqueous solution to adjust the pH to 9. The second aqueous solution was completely dialyzed with a dialysis bag with MWCO of 200 Da. The dialyzed second aqueous solution was dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 0.6 g, and the productivity rate was about 32%.

The salt of this example was subjected to high-resolution mass spectrometry detection to obtain the cation of this example, with m/z = 291.13 for (C₁₁H₁₅O₂N₈)⁺.

### Example 4

A salt of example 4 is specifically shown in formula (4). That is, Q¹ is adenine, Q² is proline, *k =* 1, and X is a hexafluorophosphate ion.

### Preparation method

The method of preparing the salt of this example is as follows, wherein:
- the first reactant was adenine;
- the second reactant was proline; and
- the first salt was ammonium phosphate, that is, a first anion was a phosphate ion.

1 g of first reactant was placed into 10 mL of 2 M hydrochloric acid at 4°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 2 g of first salt of this example was added and stirred at 4°C for 6 h to obtain a first aqueous solution including a protonated hydrochloride cation of the first reactant and the first anion.

1.5 g of second reactant was put into the first aqueous solution at 4°C and stirred gently for 48 h to obtain a second aqueous solution including the cation of this example. A 1 M sodium hydroxide solution was added to the second aqueous solution to adjust the pH to 9. The second aqueous solution was completely dialyzed with a dialysis bag with MWCO of 200 Da. The dialyzed second aqueous solution was dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 1.6 g, and the productivity rate was about 64%.

The salt of this example was subjected to high-resolution mass spectrometry detection to obtain the cation of this example, with m/z = 251.13 for (C₁₀H₁₅O₂N₆)⁺.

### Example 5

A salt of example 5 is specifically shown in formula (5). That is, Q¹ is adenine, Q² is pyrrolysine, *k =* 1, and X is a hexafluorophosphate ion.

The method of preparing the salt of this example refers to example 1. A first reactant was adenine. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 391.22 for (C₁₇H₂₇O₃N₈)⁺.

### Example 6

A salt of example 6 is specifically shown in formula (6). That is, Q¹ is adenine, Q² is serine, *k* = 1, and X is a hexafluorophosphate ion.

The method of preparing the salt of this example refers to example 1. A first reactant was adenine. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 241.10 for (C₈H₁₃O₃N₆)⁺.

### Example 7

A salt of example 7 is specifically shown in formula (7). That is, Q¹ is adenine, Q² is tryptophan, *k* = 1, and X is a hexafluorophosphate ion.

The method of preparing the salt of this example refers to example 1. A first reactant was adenine. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 340.15 for (C₁₆H₁₈O₂N₇)⁺.

### Example 8

A salt of example 8 is specifically shown in formula (8). That is, Q¹ is adenine, Q² is tyrosine, *k* = 1, and X is a hexafluorophosphate ion.

The method of preparing the salt of this example refers to example 1. A first reactant was adenine. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 317.14 for (C₁₄H₁₇O₃N₆)⁺.

### Example 9

A salt of example 9 is specifically shown in formula (9). That is, Q¹ is cytosine, Q² is alanine, k = 1, and X is a hexafluorophosphate ion.

The method of preparing the salt of this example refers to example 1. A first reactant was cytosine. A second reactant was alanine. A first salt was ammonium hexafluorophosphate, that is, a first anion was a hexafluorophosphate ion.

The salt of this example was subjected to high-resolution mass spectrometry detection to obtain the cation of this example, with m/z = 201.10 for (C₇H₁₃O₃N₄)⁺.

### Example 10

A salt of example 10 is specifically shown in formula (10). That is, Q¹ is cytosine, Q² is asparagine, *k* = 1, and X is a hexafluorophosphate ion.

The method of preparing the salt of this example refers to example 1. A first reactant was cytosine. A second reactant was asparagine. A first salt was ammonium hexafluorophosphate, that is, a first anion was a hexafluorophosphate ion.

The salt of this example was subjected to high-resolution mass spectrometry detection to obtain the cation of this example, with m/z = 244.10 for (C₈H₁₄O₄N₅)⁺.

### Example 11

A salt of example 11 is specifically shown in formula (11). That is, Q¹ is cytosine, Q² is histidine, *k =* 1, and X is a hexafluorophosphate ion.

The method of preparing the salt of this example refers to example 1. A first reactant was cytosine. A second reactant was histidine. A first salt was ammonium hexafluorophosphate, that is, a first anion was a hexafluorophosphate ion.

The salt of this example was subjected to high-resolution mass spectrometry detection to obtain the cation of this example, with m/z = 267.12 for (C₁₀H₁₅O₃N₆)⁺.

### Example 12

A salt of example 12 is specifically shown in formula (12). That is, Q¹ is cytosine, Q² is proline, *k =* 1, and X is a hexafluorophosphate ion.

The method of preparing the salt of this example refers to example 1. A first reactant was cytosine. A second reactant was proline. A first salt was ammonium phosphate, that is, a first anion was a phosphate ion.

The salt of this example was subjected to high-resolution mass spectrometry detection to obtain the cation of this example, with m/z = 227.11 for (C₉H₁₅O₃N₄)⁺.

### Example 13

A salt of example 13 is specifically shown in formula (13). That is, Q¹ is cytosine, Q² is pyrrolysine, *k =* 1, and X is a hexafluorophosphate ion.

The method of preparing the salt of this example refers to example 1. A first reactant was cytosine. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 367.21 for (C₁₆H₂₇O₄N₆)⁺.

### Example 14

A salt of example 14 is specifically shown in formula (14). That is, Q¹ is cytosine, Q² is serine, *k* = 1, and X is a hexafluorophosphate ion.

The method of preparing the salt of this example refers to example 1. A first reactant was cytosine. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 217.09 for (C₇H₁₃O₄N₄)⁺.

### Example 15

A salt of example 15 is specifically shown in formula (15). That is, Q¹ is cytosine, Q² is tryptophan, *k* = 1, and X is a hexafluorophosphate ion.

The method of preparing the salt of this example refers to example 1. A first reactant was cytosine. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 316.14 for (C₁₅H₁₈O₃N₅)⁺.

### Example 16

A salt of example 16 is specifically shown in formula (16). That is, Q¹ is cytosine, Q² is tyrosine, *k =* 1, and X is a hexafluorophosphate ion.

The method of preparing the salt of this example refers to example 1. A first reactant was cytosine. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 293.12 for (C₁₃H₁₇O₄N₄)⁺.

### Example 17

A salt of example 17 is specifically shown in formula (17). That is, Q¹ is guanine, Q² is alanine, *k =* 1, and X is a hexafluorophosphate ion.

The method of preparing the salt of this example refers to example 1. A first reactant was guanine. A second reactant was alanine. A first salt was ammonium hexafluorophosphate, that is, a first anion was a hexafluorophosphate ion.

The salt of this example was subjected to high-resolution mass spectrometry detection to obtain the cation of this example, with m/z = 241.10 for (C₈H₁₃O₃N₆)⁺.

### Example 18

A salt of example 18 is specifically shown in formula (18). That is, Q¹ is guanine, Q² is asparagine, *k* = 1, and X is a hexafluorophosphate ion.

The method of preparing the salt of this example refers to example 1. A first reactant was guanine. A second reactant was asparagine. A first salt was ammonium hexafluorophosphate, that is, a first anion was a hexafluorophosphate ion.

The salt of this example was subjected to high-resolution mass spectrometry detection to obtain the cation of this example, with m/z = 284.1 for (C₉H₁₄O₄N₇)⁺.

### Example 19

A salt of example 19 is specifically shown in formula (19). That is, Q¹ is guanine, Q² is histidine, *k =* 1, and X is a hexafluorophosphate ion.

The method of preparing the salt of this example refers to example 1. A first reactant was guanine. A second reactant was histidine. A first salt was ammonium hexafluorophosphate, that is, a first anion was a hexafluorophosphate ion.

The salt of this example was subjected to high-resolution mass spectrometry detection to obtain the cation of this example, with m/z = 307.13 for (C₁₁H₁₅O₃N₈)⁺.

### Example 20

A salt of example 20 is specifically shown in formula (20). That is, Q¹ is guanine, Q² is proline, *k =* 1, and X is a hexafluorophosphate ion.

The method of preparing the salt of this example refers to example 1. A first reactant was guanine. A second reactant was proline. A first salt was ammonium phosphate, that is, a first anion was a phosphate ion.

The salt of this example was subjected to high-resolution mass spectrometry detection to obtain the cation of this example, with m/z = 267.12 for (C₁₀H₁₅O₃N₆)⁺.

### Example 21

A salt of example 21 is specifically shown in formula (21). That is, Q¹ is guanine, Q² is pyrrolysine, *k =* 1, and X is a hexafluorophosphate ion.

The method of preparing the salt of this example refers to example 1. A first reactant was guanine. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 407.22 for (C₁₇H₂₇O₄N₈)⁺.

### Example 22

A salt of example 22 is specifically shown in formula (22). That is, Q¹ is guanine, Q² is serine, *k* = 1, and X is a hexafluorophosphate ion.

The method of preparing the salt of this example refers to example 1. A first reactant was guanine. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 257.10 for (C₈H₁₃O₄N₆)⁺.

### Example 23

A salt of example 23 is specifically shown in formula (23). That is, Q¹ is guanine, Q² is tryptophan, *k* = 1, and X is a hexafluorophosphate ion.

The method of preparing the salt of this example refers to example 1. A first reactant was guanine. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 356.15 for (C₁₆H₁₃O₃N₇)⁺.

### Example 24

A salt of example 24 is specifically shown in formula (24). That is, Q¹ is guanine, Q² is tyrosine, *k =* 1, and X is a hexafluorophosphate ion.

The method of preparing the salt of this example refers to example 1. A first reactant was guanine. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 333.13 for (C₁₄H₁₇O₄N₆)⁺.

### Example 25

A salt of example 25 is specifically shown in formula (25). That is, Q¹ is hypoxanthine, Q² is alanine, *k* = 1, and X is a hexafluorophosphate ion.

The method of preparing the salt of this example refers to example 1. A first reactant was hypoxanthine. A second reactant was alanine. A first salt was ammonium hexafluorophosphate, that is, a first anion was a hexafluorophosphate ion.

The salt of this example was subjected to high-resolution mass spectrometry detection to obtain the cation of this example, with m/z = 226.09 for (C₈H₁₂O₃N₅)⁺.

### Example 26

A salt of example 26 is specifically shown in formula (26). That is, Q¹ is hypoxanthine, Q² is asparagine, *k* = 1, and X is a hexafluorophosphate ion.

The method of preparing the salt of this example refers to example 1. A first reactant was hypoxanthine. A second reactant was asparagine. A first salt was ammonium hexafluorophosphate, that is, a first anion was a hexafluorophosphate ion.

The salt of this example was subjected to high-resolution mass spectrometry detection to obtain the cation of this example, with m/z = 269.10 for (C₉H₁₃O₄N₆)⁺.

### Example 27

A salt of example 27 is specifically shown in formula (27). That is, Q¹ is hypoxanthine, Q² is histidine, *k =* 1, and X is a hexafluorophosphate ion.

The method of preparing the salt of this example refers to example 1. A first reactant was hypoxanthine. A second reactant was histidine. A first salt was ammonium hexafluorophosphate, that is, a first anion was a hexafluorophosphate ion.

The salt of this example was subjected to high-resolution mass spectrometry detection to obtain the cation of this example, with m/z = 292.12 for (C₁₁H₁₄O₃N₇)⁺.

### Example 28

A salt of example 28 is specifically shown in formula (28). That is, Q¹ is hypoxanthine, Q² is proline, *k =* 1, and X is a hexafluorophosphate ion.

The method of preparing the salt of this example refers to example 1. A first reactant was hypoxanthine. A second reactant was proline. A first salt was ammonium phosphate, that is, a first anion was a phosphate ion.

The salt of this example was subjected to high-resolution mass spectrometry detection to obtain the cation of this example, with m/z = 252.11 for (C₁₀H₁₄O₃N₅)⁺.

### Example 29

A salt of example 29 is specifically shown in formula (29). That is, Q¹ is hypoxanthine, Q² is pyrrolysine, *k =* 1, and X is a hexafluorophosphate ion.

The method of preparing the salt of this example refers to example 1. A first reactant was hypoxanthine. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 392.20 for (C₁₇H₂₆O₄N₇)⁺.

### Example 30

A salt of example 30 is specifically shown in formula (30). That is, Q¹ is hypoxanthine, Q² is serine, *k =* 1, and X is a hexafluorophosphate ion.

The method of preparing the salt of this example refers to example 1. A first reactant was hypoxanthine. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 242.09 for (C₈H₁₂O₄N₅)⁺.

### Example 31

A salt of example 31 is specifically shown in formula (31). That is, Q¹ is hypoxanthine, Q² is tryptophan, *k* = 1, and X is a hexafluorophosphate ion.

The method of preparing the salt of this example refers to example 1. A first reactant was hypoxanthine. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 341.14 for (C₁₆H₁₇O₃N₆)⁺.

### Example 32

A salt of example 32 is specifically shown in formula (32). That is, Q¹ is hypoxanthine, Q² is tyrosine, *k =* 1, and X is a hexafluorophosphate ion.

The method of preparing the salt of this example refers to example 1. A first reactant was hypoxanthine. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 318.12 for (C₁₄H₁₆O₄N₅)⁺.

### Example 33

A salt of example 33 is specifically shown in formula (33). That is, Q¹ is thymine, Q² is alanine, *k* = 1, and X is a hexafluorophosphate ion.

The method of preparing the salt of this example refers to example 1. A first reactant was thymine. A second reactant was alanine. A first salt was ammonium hexafluorophosphate, that is, a first anion was a hexafluorophosphate ion.

The salt of this example was subjected to high-resolution mass spectrometry detection to obtain the cation of this example, with m/z = 216.10 for (C₈H₁₄O₄N₃)⁺.

### Example 34

A salt of example 34 is specifically shown in formula (34). That is, Q¹ is thymine, Q² is asparagine, *k* = 1, and X is a hexafluorophosphate ion.

The method of preparing the salt of this example refers to example 1. A first reactant was thymine. A second reactant was asparagine. A first salt was ammonium hexafluorophosphate, that is, a first anion was a hexafluorophosphate ion.

The salt of this example was subjected to high-resolution mass spectrometry detection to obtain the cation of this example, with m/z = 259.10 for (C₉H₁₅O₅N₄)⁺.

### Example 35

A salt of example 35 is specifically shown in formula (35). That is, Q¹ is thymine, Q² is histidine, *k =* 1, and X is a hexafluorophosphate ion.

The method of preparing the salt of this example refers to example 1. A first reactant was thymine. A second reactant was histidine. A first salt was ammonium hexafluorophosphate, that is, a first anion was a hexafluorophosphate ion.

The salt of this example was subjected to high-resolution mass spectrometry detection to obtain the cation of this example, with m/z = 282.12 for (C₁₁H₁₆O₄N₅)⁺.

### Example 36

A salt of example 36 is specifically shown in formula (36). That is, Q¹ is thymine, Q² is proline, *k =* 1, and X is a hexafluorophosphate ion.

The method of preparing the salt of this example refers to example 1. A first reactant was thymine. A second reactant was proline. A first salt was ammonium phosphate, that is, a first anion was a phosphate ion.

The salt of this example was subjected to high-resolution mass spectrometry detection to obtain the cation of this example, with m/z = 242.11 for (C₁₀H₁₆O₄N₃)⁺.

### Example 37

A salt of example 37 is specifically shown in formula (37). That is, Q¹ is thymine, Q² is pyrrolysine, *k =* 1, and X is a hexafluorophosphate ion.

The method of preparing the salt of this example refers to example 1. A first reactant was thymine. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 382.21 for (C₁₇H₂₈O₅N₅)⁺.

### Example 38

A salt of example 38 is specifically shown in formula (38). That is, Q¹ is thymine, Q² is serine, *k =* 1, and X is a hexafluorophosphate ion.

The method of preparing the salt of this example refers to example 1. A first reactant was thymine. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 232.09 for (C₈H₁₄O₅N₃).

### Example 39

A salt of example 39 is specifically shown in formula (39). That is, Q¹ is thymine, Q² is tryptophan, *k* = 1, and X is a hexafluorophosphate ion.

The method of preparing the salt of this example refers to example 1. A first reactant was thymine. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 331.14 for (C₁₆H₁₉O₄N₄)⁺.

### Example 40

A salt of example 40 is specifically shown in formula (40). That is, Q¹ is thymine, Q² is tyrosine, *k =* 1, and X is a hexafluorophosphate ion.

The method of preparing the salt of this example refers to example 1. A first reactant was thymine. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 308.12 for (C₁₄H₁₈O₅N₃)⁺.

### Example 41

A salt of example 41 is specifically shown in formula (41). That is, Q¹ is uracil, Q² is alanine, *k* = 1*,* and X is a hexafluorophosphate ion.

The method of preparing the salt of this example refers to example 1. A first reactant was uracil. A second reactant was alanine. A first salt was ammonium hexafluorophosphate, that is, a first anion was a hexafluorophosphate ion.

The salt of this example was subjected to high-resolution mass spectrometry detection to obtain the cation of this example, with m/z = 202.08 for (C₇H₁₂O₄N₃)⁺.

### Example 42

A salt of example 42 is specifically shown in formula (42). That is, Q¹ is uracil, Q² is asparagine, *k* = 1, and X is a hexafluorophosphate ion.

The method of preparing the salt of this example refers to example 1. A first reactant was uracil. A second reactant was asparagine. A first salt was ammonium hexafluorophosphate, that is, a first anion was a hexafluorophosphate ion.

The salt of this example was subjected to high-resolution mass spectrometry detection to obtain the cation of this example, with m/z = 245.09 for (C₈H₁₃O₅N₄)⁺.

### Example 43

A salt of example 43 is specifically shown in formula (43). That is, Q¹ is uracil, Q² is histidine, *k* = 1*,* and X is a hexafluorophosphate ion.

The method of preparing the salt of this example refers to example 1. A first reactant was uracil. A second reactant was histidine. A first salt was ammonium hexafluorophosphate, that is, a first anion was a hexafluorophosphate ion.

The salt of this example was subjected to high-resolution mass spectrometry detection to obtain the cation of this example, with m/z = 268.10 for (C₁₀H₁₄O₄N₅)⁺.

### Example 44

A salt of example 44 is specifically shown in formula (44). That is, Q¹ is uracil, Q² is proline, *k* = 1*,* and X is a hexafluorophosphate ion.

The method of preparing the salt of this example refers to example 1. A first reactant was uracil. A second reactant was proline. A first salt was ammonium phosphate, that is, a first anion was a phosphate ion.

The salt of this example was subjected to high-resolution mass spectrometry detection to obtain the cation of this example, with m/z = 228.10 for (C₉H₁₄O₄N₃)⁺.

### Example 45

A salt of example 45 is specifically shown in formula (45). That is, Q¹ is uracil, Q² is pyrrolysine, *k =* 1, and X is a hexafluorophosphate ion.

The method of preparing the salt of this example refers to example 1. A first reactant was uracil. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 368.19 for (C₁₆H₂₆O₅N₅)⁺.

### Example 46

A salt of example 46 is specifically shown in formula (46). That is, Q¹ is uracil, Q² is serine, *k* = 1*,* and X is a hexafluorophosphate ion.

The method of preparing the salt of this example refers to example 1. A first reactant was uracil. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 218.08 for (C₇H₁₂O₅N₃) .

### Example 47

A salt of example 47 is specifically shown in formula (47). That is, Q¹ is uracil, Q² is tryptophan, *k* = 1, and X is a hexafluorophosphate ion.

The method of preparing the salt of this example refers to example 1. A first reactant was uracil. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 317.12 for (C₁₅H₁₇O₄N₄)⁺.

### Example 48

A salt of example 48 is specifically shown in formula (48). That is, Q¹ is uracil, Q² is tyrosine, *k* = 1*,* and X is a hexafluorophosphate ion.

The method of preparing the salt of this example refers to example 1. A first reactant was uracil. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 294.11 for (C₁₃H₁₆O₅N₃).

### Example 49

A salt of example 49 is specifically shown in formula (49). That is, Q¹ is cytidine, Q² is alanine, *k* = 1, and X is a hexafluorophosphate ion.

The method of preparing the salt of this example refers to example 1. A first reactant was cytidine. A second reactant was alanine. A first salt was ammonium hexafluorophosphate, that is, a first anion was a hexafluorophosphate ion.

The salt of this example was subjected to high-resolution mass spectrometry detection to obtain the cation of this example, with m/z = 333.14 for (C₁₂H₂₁O₇N₄)⁺.

### Example 50

A salt of example 50 is specifically shown in formula (50). That is, Q¹ is cytidine, Q² is asparagine, *k* = 1, and X is a hexafluorophosphate ion.

The method of preparing the salt of this example refers to example 1. A first reactant was cytidine. A second reactant was asparagine. A first salt was ammonium hexafluorophosphate, that is, a first anion was a hexafluorophosphate ion.

The salt of this example was subjected to high-resolution mass spectrometry detection to obtain the cation of this example, with m/z = 376.15 for (C₁₃H₂₂O₈N₅)⁺.

### Example 51

A salt of example 51 is specifically shown in formula (51). That is, Q¹ is cytidine, Q² is histidine, *k =* 1, and X is a hexafluorophosphate ion.

The method of preparing the salt of this example refers to example 1. A first reactant was cytidine. A second reactant was histidine. A first salt was ammonium hexafluorophosphate, that is, a first anion was a hexafluorophosphate ion.

The salt of this example was subjected to high-resolution mass spectrometry detection to obtain the cation of this example, with m/z = 399.16 for (C₁₅H₂₃O₇N₆)⁺.

### Example 52

A salt of example 52 is specifically shown in formula (52). That is, Q¹ is cytidine, Q² is proline, *k* = 1*,* and X is a hexafluorophosphate ion.

The method of preparing the salt of this example refers to example 1. A first reactant was cytidine. A second reactant was proline. A first salt was ammonium phosphate, that is, a first anion was a phosphate ion.

The salt of this example was subjected to high-resolution mass spectrometry detection to obtain the cation of this example, with m/z = 359.16 for (C₁₄H₂₃O₇N₄)⁺.

### Example 53

A salt of example 53 is specifically shown in formula (53). That is, Q¹ is cytidine, Q² is pyrrolysine, *k =* 1, and X is a hexafluorophosphate ion.

The method of preparing the salt of this example refers to example 1. A first reactant was cytidine. A second reactant was pyrrolysine. A first salt was ammonium hexafluorophosphate, that is, a first anion was a hexafluorophosphate ion.

The salt of this example was subjected to high-resolution mass spectrometry detection to obtain the cation of this example, with m/z = 499.25 for (C₂₁H₃₅O₈N₆)⁺.

### Example 54

A salt of example 54 is specifically shown in formula (54). That is, Q¹ is cytidine, Q² is serine, *k* = 1*,* and X is a hexafluorophosphate ion.

The method of preparing the salt of this example refers to example 1. A first reactant was cytidine. A second reactant was serine. A first salt was ammonium hexafluorophosphate, that is, a first anion was a hexafluorophosphate ion.

The salt of this example was subjected to high-resolution mass spectrometry detection to obtain the cation of this example, with m/z = 349.14 for (C₁₂H₂₁O₈N₄)⁺.

### Example 55

A salt of example 55 is specifically shown in formula (55). That is, Q¹ is cytidine, Q² is tryptophan, *k* = 1, and X is a hexafluorophosphate ion.

The method of preparing the salt of this example refers to example 1. A first reactant was cytidine. A second reactant was tryptophan. A first salt was ammonium hexafluorophosphate, that is, a first anion was a hexafluorophosphate ion.

The salt of this example was subjected to high-resolution mass spectrometry detection to obtain the cation of this example, with m/z = 448.18 for (C₂₀H₂₆O₇N₅)⁺.

### Example 56

A salt of example 56 is specifically shown in formula (56). That is, Q¹ is cytidine, Q² is tyrosine, *k =* 1, and X is a hexafluorophosphate ion.

The method of preparing the salt of this example refers to example 1. A first reactant was cytidine. A second reactant was phenylalanine. A first salt was ammonium hexafluorophosphate, that is, a first anion was a hexafluorophosphate ion.

The salt of this example was subjected to high-resolution mass spectrometry detection to obtain the cation of this example, with m/z = 425.17 for (C₁₈H₂₅O₈N₄)⁺.

### Example 57

A salt of example 57 is specifically shown in formula (57). That is, Q¹ is histidine, Q² is adenine, *k =* 1, and X is a hexafluorophosphate ion.

The method of preparing the salt of this example refers to example 1. A first reactant was histidine. A second reactant was adenine. A first salt was ammonium hexafluorophosphate, that is, a first anion was a hexafluorophosphate ion.

The salt of this example was subjected to high-resolution mass spectrometry detection to obtain the cation of this example, with m/z = 291.13 for (C₁₁H₁₅O₂N₈)⁺.

### Example 58

A salt of example 58 is specifically shown in formula (58). That is, Q¹ is histidine, Q² is cytosine, *k =* 1, and X is a hexafluorophosphate ion.

The method of preparing the salt of this example refers to example 1. A first reactant was histidine. A second reactant was cytosine. A first salt was ammonium hexafluorophosphate, that is, a first anion was a hexafluorophosphate ion.

The salt of this example was subjected to high-resolution mass spectrometry detection to obtain the cation of this example, with m/z = 267.12 for (C₁₀H₁₅O₃N₆)⁺.

### Example 59

A salt of example 59 is specifically shown in formula (59). That is, Q¹ is histidine, Q² is guanine, *k* = 1, and X is a hexafluorophosphate ion.

The method of preparing the salt of this example refers to example 1. A first reactant was histidine. A second reactant was guanine. A first salt was ammonium hexafluorophosphate, that is, a first anion was a hexafluorophosphate ion.

The salt of this example was subjected to high-resolution mass spectrometry detection to obtain the cation of this example, with m/z = 307.13 for (C₁₁H₁₅O₃N₈)⁺.

### Example 60

A salt of example 60 is specifically shown in formula (60). That is, Q¹ is histidine, Q² is hypoxanthine, *k* = 1, and X is a hexafluorophosphate ion.

The method of preparing the salt of this example refers to example 1. A first reactant was histidine. A second reactant was hypoxanthine. A first salt was ammonium hexafluorophosphate, that is, a first anion was a hexafluorophosphate ion.

The salt of this example was subjected to high-resolution mass spectrometry detection to obtain the cation of this example, with m/z = 292.12 for (C₁₁H₁₄0₃N₇)⁺.

### Example 61

A salt of example 61 is specifically shown in formula (61). That is, Q¹ is histidine, Q² is thymine, *k =* 1, and X is a hexafluorophosphate ion.

The method of preparing the salt of this example refers to example 1. A first reactant was histidine. A second reactant was thymine. A first salt was ammonium hexafluorophosphate, that is, a first anion was a hexafluorophosphate ion.

The salt of this example was subjected to high-resolution mass spectrometry detection to obtain the cation of this example, with m/z = 282.12 for (C₁₁H₁₆O₄N₅)⁺.

### Example 62

A salt of example 62 is specifically shown in formula (62). That is, Q¹ is histidine, Q² is uracil, *k =* 1, and X is a hexafluorophosphate ion.

The method of preparing the salt of this example refers to example 1. A first reactant was histidine. A second reactant was uracil. A first salt was ammonium hexafluorophosphate, that is, a first anion was a hexafluorophosphate ion.

The salt of this example was subjected to high-resolution mass spectrometry detection to obtain the cation of this example, with m/z = 268.10 for (C₁₀H₁₄O₄N₅)⁺.

### Example 63

A salt of example 63 is specifically shown in formula (63). That is, Q¹ is histidine, Q² is guanosine, *k* = 1, and X is a hexafluorophosphate ion.

The method of preparing the salt of this example refers to example 1. A first reactant was histidine. A second reactant was guanosine. A first salt was ammonium hexafluorophosphate, that is, a first anion was a hexafluorophosphate ion.

The salt of this example was subjected to high-resolution mass spectrometry detection to obtain the cation of this example, with m/z = 423.17 for (C₁₆H₂₃O₆N₈)⁺.

### Example 64

A salt of example 64 is specifically shown in formula (64). That is, Q¹ is histidine, Q² is cytidine, *k* = 1, and X is a hexafluorophosphate ion.

The method of preparing the salt of this example refers to example 1. A first reactant was histidine. A second reactant was cytidine. A first salt was ammonium hexafluorophosphate, that is, a first anion was a hexafluorophosphate ion.

The salt of this example was subjected to high-resolution mass spectrometry detection to obtain the cation of this example, with m/z = 399.16 for (C₁₅H₂₃O₇N₆)⁺.

### Example 65

A salt of example 65 is specifically shown in formula (65). That is, Q¹ is proline, Q² is adenine, *k* = 1*,* and X is a hexafluorophosphate ion.

The method of preparing the salt of this example refers to example 1. A first reactant was proline. A second reactant was adenine. A first salt was ammonium hexafluorophosphate, that is, a first anion was a hexafluorophosphate ion.

The salt of this example was subjected to high-resolution mass spectrometry detection to obtain the cation of this example, with m/z = 251.13 for (C₁₀H₁₅O₂N₆)⁺.

### Example 66

A salt of example 66 is specifically shown in formula (66). That is, Q¹ is proline, Q² is cytosine, *k =* 1, and X is a hexafluorophosphate ion.

The method of preparing the salt of this example refers to example 1. A first reactant was proline. A second reactant was cytosine. A first salt was ammonium hexafluorophosphate, that is, a first anion was a hexafluorophosphate ion.

The salt of this example was subjected to high-resolution mass spectrometry detection to obtain the cation of this example, with m/z = 227.11 for (C₉H₁₅O₃N₄)⁺.

### Example 67

A salt of example 67 is specifically shown in formula (67). That is, Q¹ is proline, Q² is guanine, *k* = 1*,* and X is a hexafluorophosphate ion.

The method of preparing the salt of this example refers to example 1. A first reactant was proline. A second reactant was guanine. A first salt was ammonium hexafluorophosphate, that is, a first anion was a hexafluorophosphate ion.

The salt of this example was subjected to high-resolution mass spectrometry detection to obtain the cation of this example, with m/z = 267.12 for (C₁₀H₁₅O₃N₆)⁺.

### Example 68

A salt of example 68 is specifically shown in formula (68). That is, Q¹ is proline, Q² is hypoxanthine, *k* = 1, and X is a hexafluorophosphate ion.

The method of preparing the salt of this example refers to example 1. A first reactant was proline. A second reactant was hypoxanthine. A first salt was ammonium hexafluorophosphate, that is, a first anion was a hexafluorophosphate ion.

The salt of this example was subjected to high-resolution mass spectrometry detection to obtain the cation of this example, with m/z = 252.11 for (C₁₀H₁₄O₃N₅)⁺.

### Example 69

A salt of example 69 is specifically shown in formula (69). That is, Q¹ is proline, Q² is thymine, *k* = 1*,* and X is a hexafluorophosphate ion.

The method of preparing the salt of this example refers to example 1. A first reactant was proline. A second reactant was thymine. A first salt was ammonium hexafluorophosphate, that is, a first anion was a hexafluorophosphate ion.

The salt of this example was subjected to high-resolution mass spectrometry detection to obtain the cation of this example, with m/z = 242.11 for (C₁₀H₁₆O₄N₃)⁺.

### Example 70

A salt of example 70 is specifically shown in formula (70). That is, Q¹ is proline, Q² is uracil, *k* = 1*,* and X is a hexafluorophosphate ion.

The method of preparing the salt of this example refers to example 1. A first reactant was proline. A second reactant was uracil. A first salt was ammonium hexafluorophosphate, that is, a first anion was a hexafluorophosphate ion.

The salt of this example was subjected to high-resolution mass spectrometry detection to obtain the cation of this example, with m/z = 228.10 for (C₉H₁₄O₄N₃)⁺.

### Example 71

A salt of example 71 is specifically shown in formula (71). That is, Q¹ is proline, Q² is guanosine, *k* = 1, and X is a hexafluorophosphate ion.

The method of preparing the salt of this example refers to example 1. A first reactant was proline. A second reactant was guanosine. A first salt was ammonium hexafluorophosphate, that is, a first anion was a hexafluorophosphate ion.

The salt of this example was subjected to high-resolution mass spectrometry detection to obtain the cation of this example, with m/z = 383.17 for (C₁₃H₂₃O₆N₆)⁺.

### Example 72

A salt of example 72 is specifically shown in formula (72). That is, Q¹ is proline, Q² is cytidine, *k* = 1*,* and X is a hexafluorophosphate ion.

The method of preparing the salt of this example refers to example 1. A first reactant was proline. A second reactant was cytidine. A first salt was ammonium hexafluorophosphate, that is, a first anion was a hexafluorophosphate ion.

The salt of this example was subjected to high-resolution mass spectrometry detection to obtain the cation of this example, with m/z = 359.16 for (C₁₄H₂₃O₇N₄)⁺.

### Example 73

A salt of example 73 is specifically shown in formula (73). That is, Q¹ is tryptophan, Q² is adenine, *k =* 1, and X is a hexafluorophosphate ion.

The method of preparing the salt of this example refers to example 1. A first reactant was tryptophan. A second reactant was adenine. A first salt was ammonium hexafluorophosphate, that is, a first anion was a hexafluorophosphate ion.

The salt of this example was subjected to high-resolution mass spectrometry detection to obtain the cation of this example, with m/z = 340.15 for (C₁₆H₁₈O₂N₇)⁺.

### Example 74

A salt of example 74 is specifically shown in formula (74). That is, Q¹ is tryptophan, Q² is cytosine, *k =* 1, and X is a hexafluorophosphate ion.

The method of preparing the salt of this example refers to example 1. A first reactant was tryptophan. A second reactant was cytosine. A first salt was ammonium hexafluorophosphate, that is, a first anion was a hexafluorophosphate ion.

The salt of this example was subjected to high-resolution mass spectrometry detection to obtain the cation of this example, with m/z = 316.14 for (C₁₃H₁₈O₃N₅)⁺.

### Example 75

A salt of example 75 is specifically shown in formula (75). That is, Q¹ is tryptophan, Q² is guanine, *k* = 1, and X is a hexafluorophosphate ion.

The method of preparing the salt of this example refers to example 1. A first reactant was tryptophan. A second reactant was guanine. A first salt was ammonium hexafluorophosphate, that is, a first anion was a hexafluorophosphate ion.

The salt of this example was subjected to high-resolution mass spectrometry detection to obtain the cation of this example, with m/z = 356.15 for (C₁₆H₁₈O₃N₇)⁺.

### Example 76

A salt of example 76 is specifically shown in formula (76). That is, Q¹ is tryptophan, Q² is hypoxanthine, *k* = 1, and X is a hexafluorophosphate ion.

The method of preparing the salt of this example refers to example 1. A first reactant was tryptophan. A second reactant was hypoxanthine. A first salt was ammonium hexafluorophosphate, that is, a first anion was a hexafluorophosphate ion.

The salt of this example was subjected to high-resolution mass spectrometry detection to obtain the cation of this example, with m/z = 341.14 for (C₁₆H₁₇O₃N₆)⁺.

### Example 77

A salt of example 77 is specifically shown in formula (77). That is, Q¹ is tryptophan, Q² is thymine, *k =* 1, and X is a hexafluorophosphate ion.

The method of preparing the salt of this example refers to example 1. A first reactant was tryptophan. A second reactant was thymine. A first salt was ammonium hexafluorophosphate, that is, a first anion was a hexafluorophosphate ion.

The salt of this example was subjected to high-resolution mass spectrometry detection to obtain the cation of this example, with m/z = 331.14 for (C₁₆H₁₉O₄N₄)⁺.

### Example 78

A salt of example 78 is specifically shown in formula (78). That is, Q¹ is tryptophan, Q² is uracil, *k =* 1, and X is a hexafluorophosphate ion.

The method of preparing the salt of this example refers to example 1. A first reactant was tryptophan. A second reactant was uracil. A first salt was ammonium hexafluorophosphate, that is, a first anion was a hexafluorophosphate ion.

The salt of this example was subjected to high-resolution mass spectrometry detection to obtain the cation of this example, with m/z = 317.12 for (C₁₃H₁₇O₄N₄)⁺.

### Example 79

A salt of example 79 is specifically shown in formula (79). That is, Q¹ is tryptophan, Q² is guanosine, *k* = 1, and X is a hexafluorophosphate ion.

The method of preparing the salt of this example refers to example 1. A first reactant was tryptophan. A second reactant was guanosine. A first salt was ammonium hexafluorophosphate, that is, a first anion was a hexafluorophosphate ion.

The salt of this example was subjected to high-resolution mass spectrometry detection to obtain the cation of this example, with m/z = 472.19 for (C₂₁H₂₆O₆N₇)⁺.

### Example 80

A salt of example 80 is specifically shown in formula (80). That is, Q¹ is tryptophan, Q² is cytidine, *k* = 1, and X is a hexafluorophosphate ion.

The method of preparing the salt of this example refers to example 1. A first reactant was tryptophan. A second reactant was cytidine. A first salt was ammonium hexafluorophosphate, that is, a first anion was a hexafluorophosphate ion.

The salt of this example was subjected to high-resolution mass spectrometry detection to obtain the cation of this example, with m/z = 448.18 for (C₂₀H₂₆O₇N₅)⁺.

### Example 81

A salt of example 81 is specifically shown in formula (81). That is, Q¹ is pyrrolysine, Q² is adenine, *k =* 1, and X is a hexafluorophosphate ion.

The method of preparing the salt of this example refers to example 1. A first reactant was pyrrolysine. A second reactant was adenine. A first salt was ammonium hexafluorophosphate, that is, a first anion was a hexafluorophosphate ion.

The salt of this example was subjected to high-resolution mass spectrometry detection to obtain the cation of this example, with m/z = 391.22 for (C₁₇H₂₇O₃N₈)⁺.

### Example 82

A salt of example 82 is specifically shown in formula (82). That is, Q¹ is pyrrolysine, Q² is cytosine, *k =* 1, and X is a hexafluorophosphate ion.

The method of preparing the salt of this example refers to example 1. A first reactant was pyrrolysine. A second reactant was cytosine. A first salt was ammonium hexafluorophosphate, that is, a first anion was a hexafluorophosphate ion.

The salt of this example was subjected to high-resolution mass spectrometry detection to obtain the cation of this example, with m/z = 367.21 for (C₁₆H₂₇O₄N₆)⁺.

### Example 83

A salt of example 83 is specifically shown in formula (83). That is, Q¹ is pyrrolysine, Q² is guanine, *k* = 1, and X is a hexafluorophosphate ion.

The method of preparing the salt of this example refers to example 1. A first reactant was pyrrolysine. A second reactant was guanine. A first salt was ammonium hexafluorophosphate, that is, a first anion was a hexafluorophosphate ion.

The salt of this example was subjected to high-resolution mass spectrometry detection to obtain the cation of this example, with m/z = 407.22 for (C₁₇H₂₇O₄N₈)⁺.

### Example 84

A salt of example 84 is specifically shown in formula (84). That is, Q¹ is pyrrolysine, Q² is hypoxanthine, *k* = 1, and X is a hexafluorophosphate ion.

The method of preparing the salt of this example refers to example 1. A first reactant was pyrrolysine. A second reactant was hypoxanthine. A first salt was ammonium hexafluorophosphate, that is, a first anion was a hexafluorophosphate ion.

The salt of this example was subjected to high-resolution mass spectrometry detection to obtain the cation of this example, with m/z = 392.20 for (C₁₇H₂₆O₄N₇)⁺.

### Example 85

A salt of example 85 is specifically shown in formula (85). That is, Q¹ is pyrrolysine, Q² is thymine, *k =* 1, and X is a hexafluorophosphate ion.

The method of preparing the salt of this example refers to example 1. A first reactant was pyrrolysine. A second reactant was thymine. A first salt was ammonium hexafluorophosphate, that is, a first anion was a hexafluorophosphate ion.

The salt of this example was subjected to high-resolution mass spectrometry detection to obtain the cation of this example, with m/z = 382.21 for (C₁₇H₂₈O₅N₅)⁺.

### Example 86

A salt of example 86 is specifically shown in formula (86). That is, Q¹ is pyrrolysine, Q² is uracil, *k =* 1, and X is a hexafluorophosphate ion.

The method of preparing the salt of this example refers to example 1. A first reactant was pyrrolysine. A second reactant was uracil. A first salt was ammonium hexafluorophosphate, that is, a first anion was a hexafluorophosphate ion.

The salt of this example was subjected to high-resolution mass spectrometry detection to obtain the cation of this example, with m/z = 368.19 for (C₁₆H₂₆O₅N₅)⁺.

### Example 87

A salt of example 87 is specifically shown in formula (87). That is, Q¹ is adenine, Q² is tryptophan, *k* = 1, and X is an acetate ion.

### Preparation method

The method of preparing the salt of this example is as follows, wherein:
- the first reactant was adenine;
- the second reactant was tryptophan; and
- the first salt was ammonium acetate, that is, a first anion was an acetate ion.

1 g of first reactant was placed into 10 mL of 2 M hydrochloric acid at 25°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 4 g of first salt of this example was added and stirred at 25°C for 6 h to obtain a first aqueous solution including a protonated hydrochloride cation of the first reactant and the first anion.

0.89 g of second reactant was put into the first aqueous solution at 25°C and stirred gently for 24 h to obtain a second aqueous solution including the cation of this example. The second aqueous solution was completely dialyzed with a dialysis bag with MWCO of 200 Da, and then dried to obtain a first intermediate. The first intermediate was dissolved in 10 mL of ultrapure water, dilute ammonia water was added to adjust the pH to 9, and a large amount of beige precipitates were precipitated, followed by filtration. The filter cake was washed twice with water and dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 1.6 g, and the productivity rate was about 85%.

The salt of this example was subjected to high-resolution mass spectrometry detection to obtain the cation of this example, with m/z = 340.15 for (C₁₆H₁₈O₂N₇)⁺.

### Example 88

A salt of example 88 is specifically shown in formula (88). That is, Q¹ is adenine, Q² is tryptophan, *k* = 2, and X is a succinate ion.

### Preparation method

The method of preparing the salt of this example is as follows, wherein:
- the first reactant was adenine;
- the second reactant was tryptophan; and
- the first salt was ammonium succinate, that is, a first anion was a succinate ion.

1 g of first reactant was placed into 10 mL of 2 M hydrochloric acid at 37°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 4 g of first salt of this example was added and stirred at 37°C for 12 h to obtain a first aqueous solution including a protonated hydrochloride cation of the first reactant and the first anion.

0.89 g of second reactant was put into the first aqueous solution at 37°C and stirred gently for 36 h to obtain a second aqueous solution including the cation of this example. A 1 M sodium hydroxide solution was added to the second aqueous solution to adjust the pH to 9. The second aqueous solution was completely dialyzed with a dialysis bag with MWCO of 200 Da. The dialyzed second aqueous solution was dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 1.2 g, and the productivity rate was about 63%.

The salt of this example was subjected to high-resolution mass spectrometry detection to obtain the cation of this example, with m/z = 340.15 for (C₁₆H₁₈O₂N₇)⁺.

### Example 89

A salt of example 89 is specifically shown in formula (89). That is, Q¹ is adenine, Q² is tryptophan, *k* = 2, and X is a sulfate ion.

### Preparation method

The method of preparing the salt of this example is as follows, wherein:
- the first reactant was adenine;
- the second reactant was tryptophan; and
- the first salt was ammonium sulfate, that is, a first anion was a sulfate ion.

1 g of first reactant was placed into 10 mL of 2 M hydrochloric acid at 60°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 2 g of first salt of this example was added and stirred at 60°C for 6 h to obtain a first aqueous solution including a protonated hydrochloride cation of the first reactant and the first anion.

0.65 g of second reactant was put into the first aqueous solution at 60°C and stirred gently for 48 h to obtain a second aqueous solution including the cation of this example. A 1 M sodium hydroxide solution was added to the second aqueous solution to adjust the pH to 9. The second aqueous solution was completely dialyzed with a dialysis bag with MWCO of 200 Da. The dialyzed second aqueous solution was dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 0.6 g, and the productivity rate was about 32%.

The salt of this example was subjected to high-resolution mass spectrometry detection to obtain the cation of this example, with m/z = 340.15 for (C₁₆H₁₈O₂N₇)⁺.

### Example 90

A salt of example 90 is specifically shown in formula (90). That is, Q¹ is adenine, Q² is tryptophan, *k* = 3, and X is a phosphate ion.

### Preparation method

The method of preparing the salt of this example is as follows, wherein:
- the first reactant was adenine;
- the second reactant was tryptophan; and
- the first salt was ammonium phosphate, that is, a first anion was a phosphate ion.

1 g of first reactant was placed into 10 mL of 2 M hydrochloric acid at 4°C and stirred intensely until the first reactant was completely dissolved. The solution was heated to 85°C and kept at this temperature for 30 min, and concentrated under reduced pressure to 2 mL. After the solution was cooled, crystals were precipitated, washed 3 times with an ethanol solution, and dried to obtain a hydrochloride of the first reactant.

0.8 g of hydrochloride of the first reactant was placed into 10 mL of ultrapure water and stirred intensely until the hydrochloride of the first reactant was completely dissolved, and 2 g of first salt of this example was added and stirred at 4°C for 6 h to obtain a first aqueous solution including a protonated hydrochloride cation of the first reactant and the first anion.

1.5 g of second reactant was put into the first aqueous solution at 4°C and stirred gently for 48 h to obtain a second aqueous solution including the cation of this example. A 1 M sodium hydroxide solution was added to the second aqueous solution to adjust the pH to 9. The second aqueous solution was completely dialyzed with a dialysis bag with MWCO of 200 Da. The dialyzed second aqueous solution was dried to obtain a product, *i.e.,* the salt of this example.

### Detection of product or intermediate

The hydrochloride of the first reactant in this example was white crystals, with a yield of about 0.8 g and a productivity rate of about 80%.

The yield of the salt of this example was about 1.6 g, and the productivity rate was about 64%.

The salt of this example was subjected to high-resolution mass spectrometry detection to obtain the cation of this example, with m/z = 340.15 for (C₁₆H₁₈ON₇)⁺.

### STRUCTURAL FORMULAE OF THE EXAMPLES

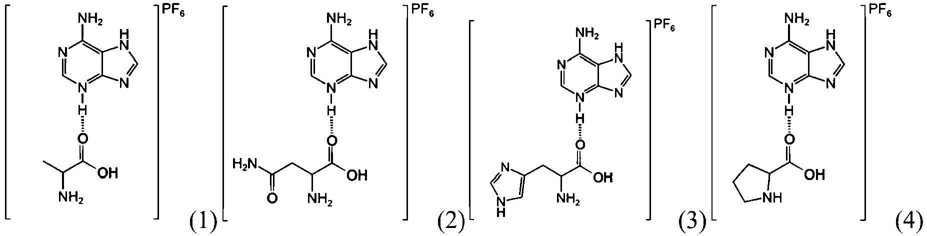

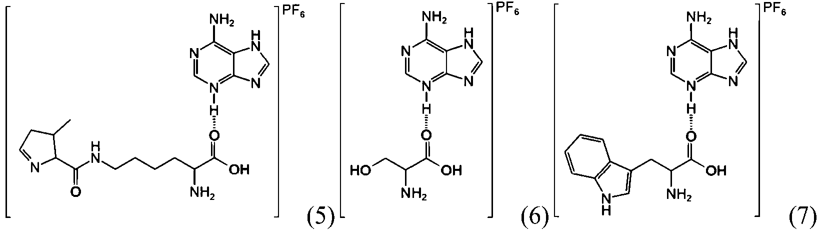

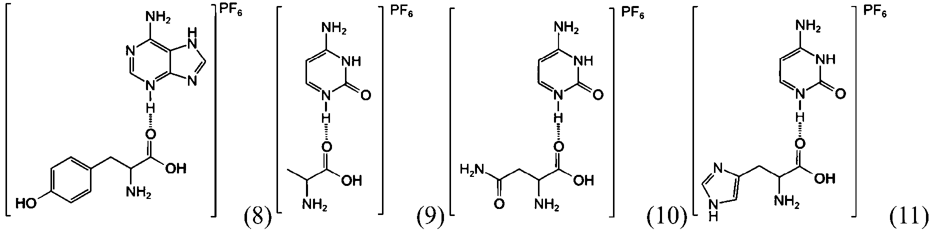

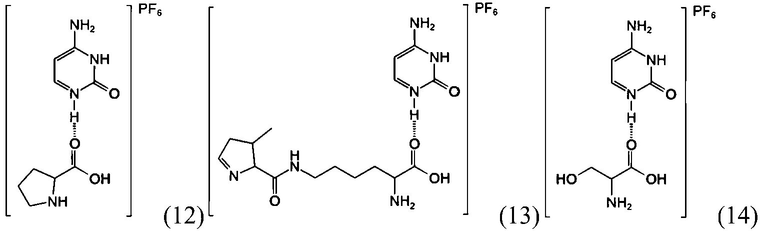

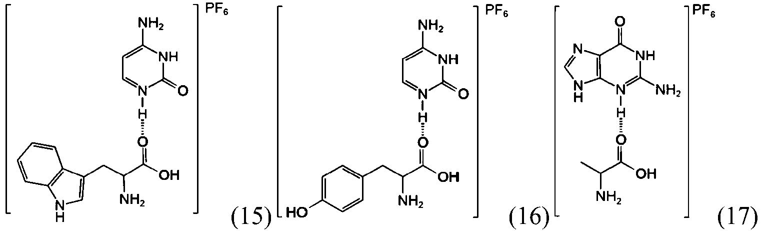

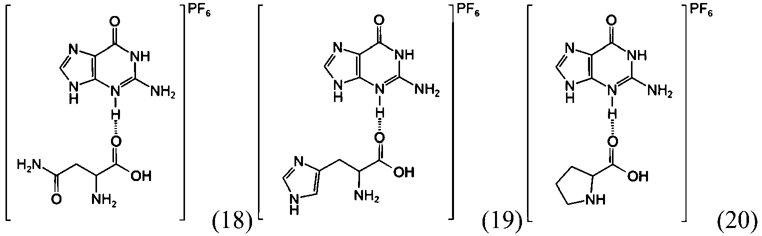

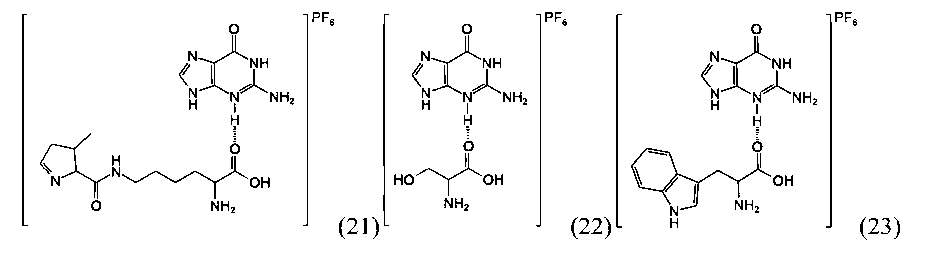

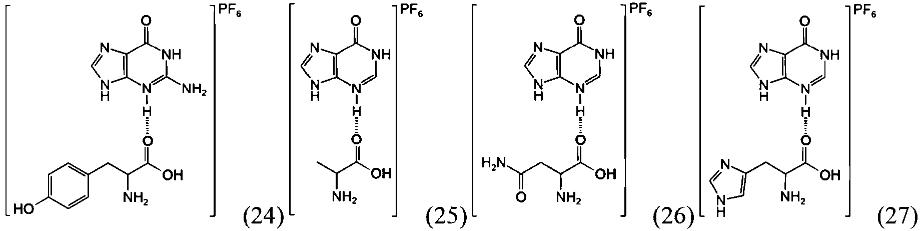

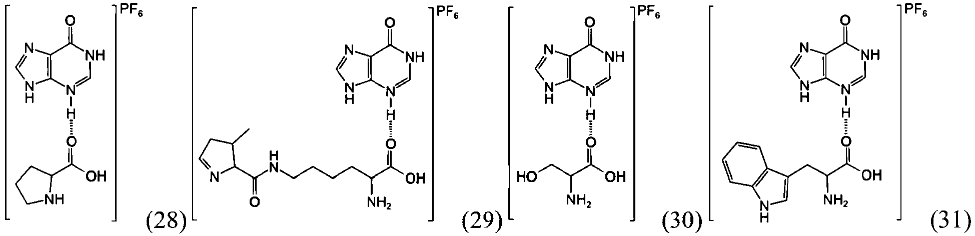

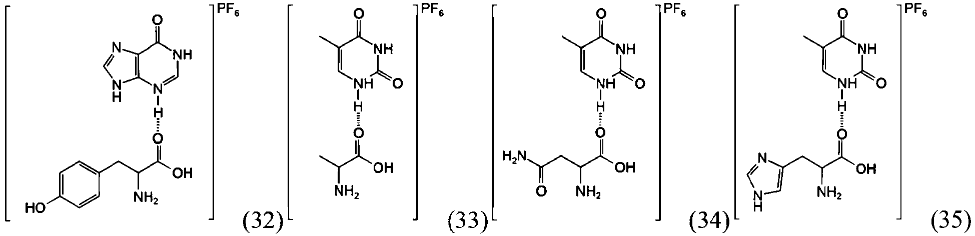

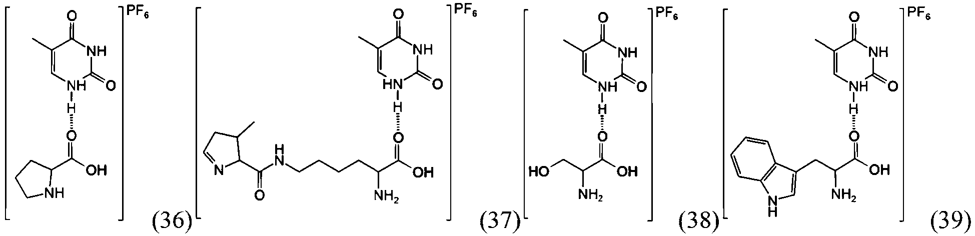

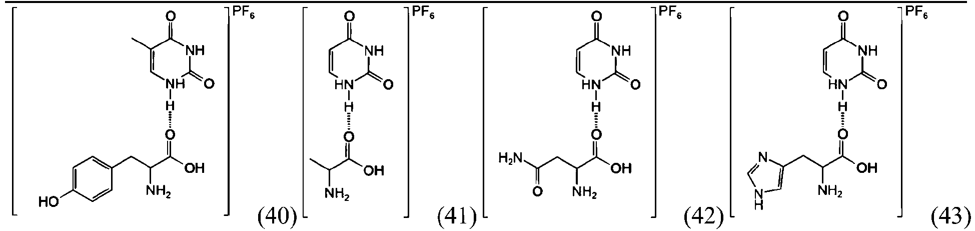

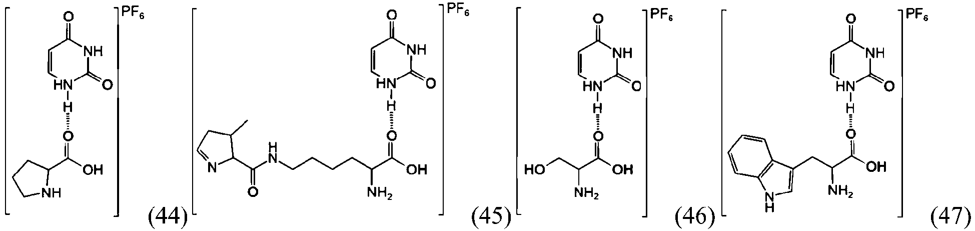

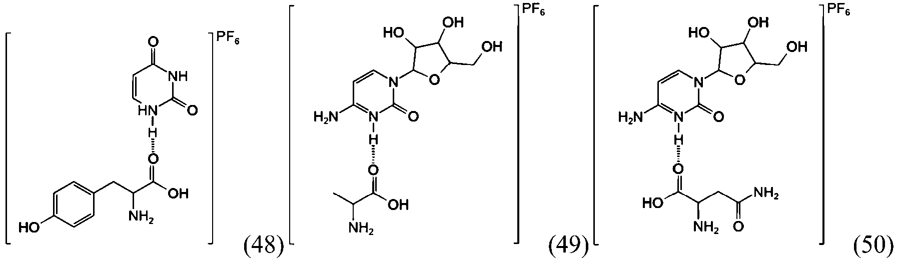

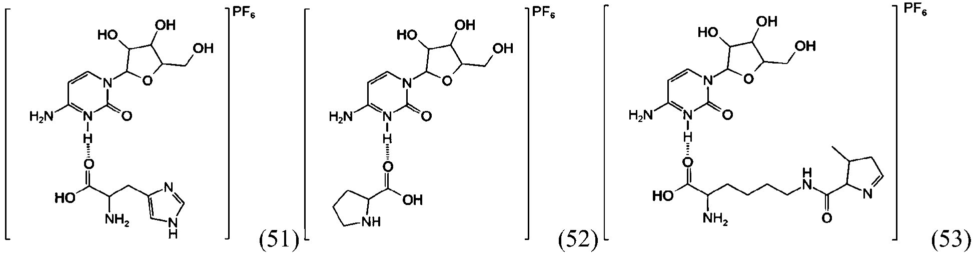

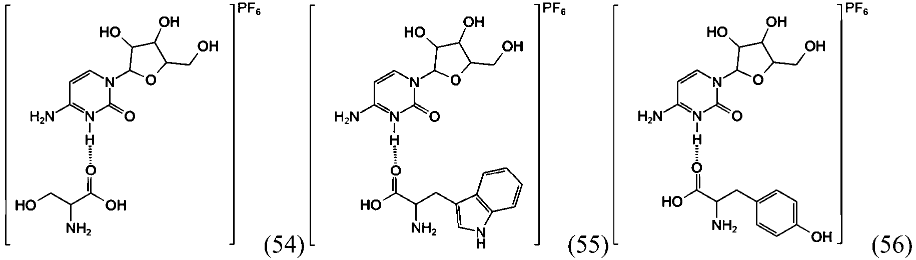

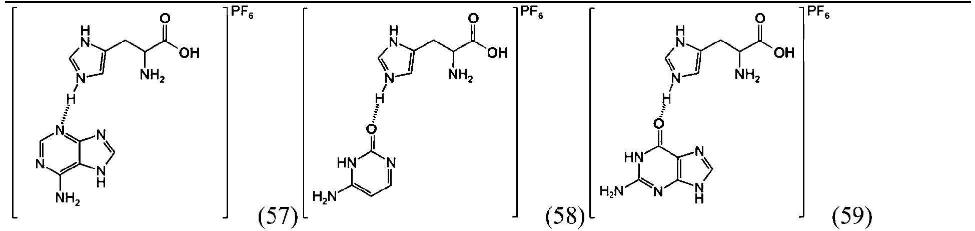

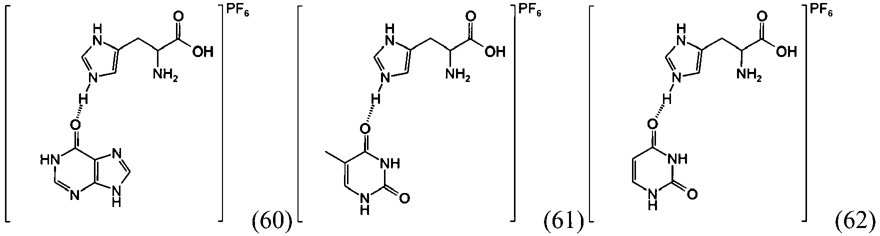

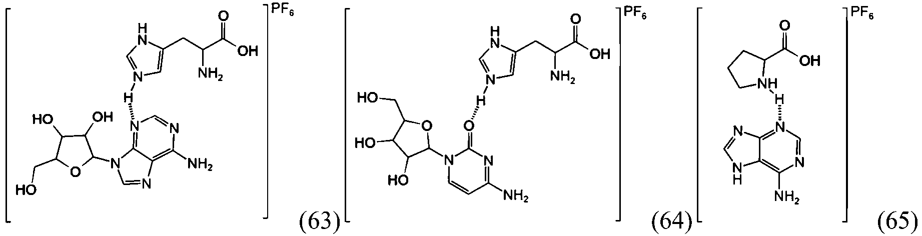

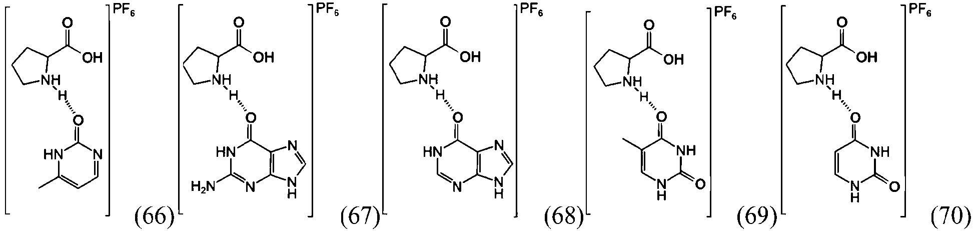

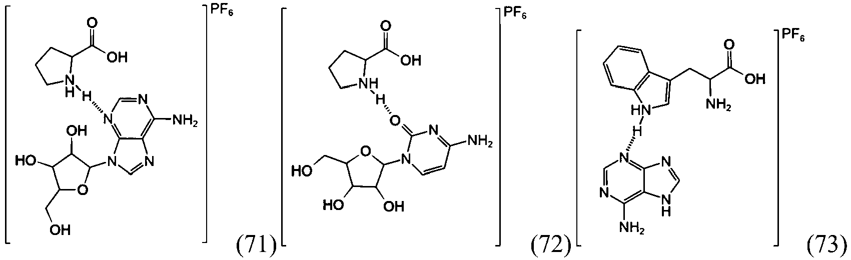

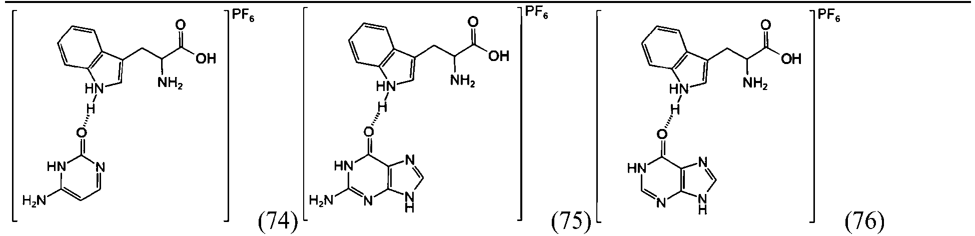

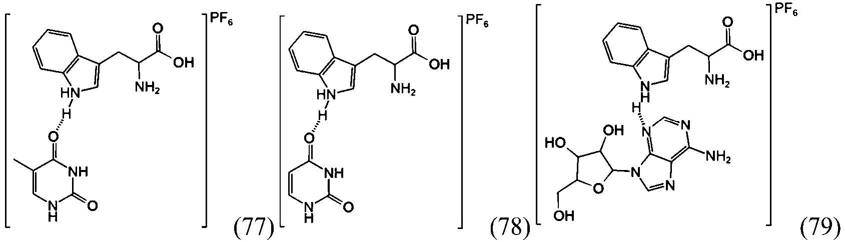

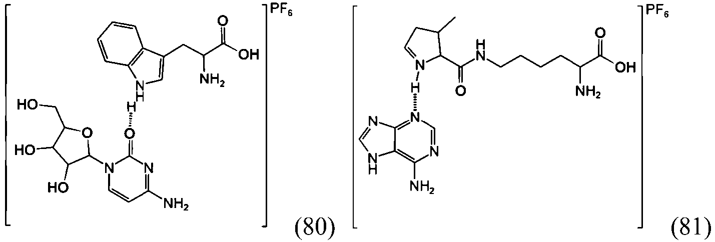

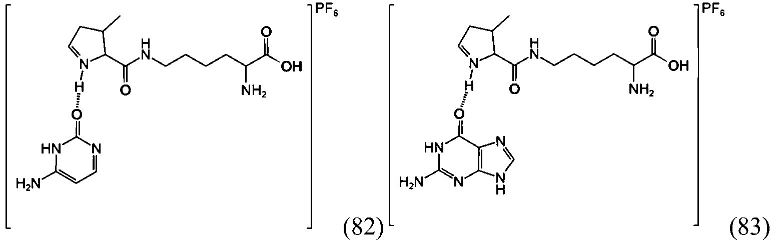

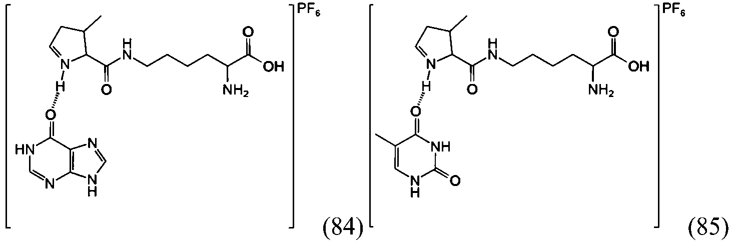

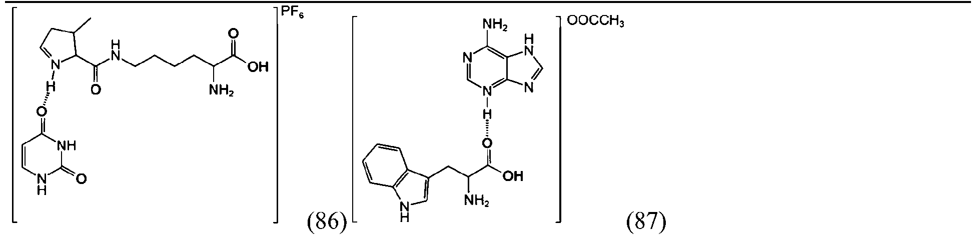

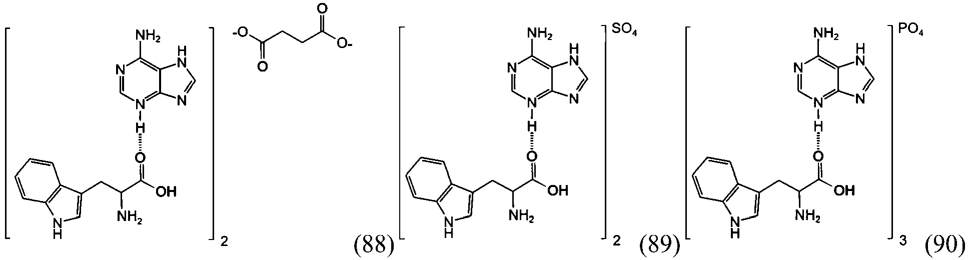

### Ethanol-induced damage model of gastric mucosal epithelial cells

Chronic alcoholics are prone to gastric mucosal damage due to long-term alcohol damage. In this experiment, a gastric mucosal epithelial cell damage model was established at a cellular level with ethanol, the salt of the present application or another control compound was added, and the technical effects of the non-covalent dimer cation of the present application in the gastric mucosal epithelial cell damage model were obtained

The salts of example 51 and example 57 were taken as experimental compounds, and glycyrrhetinic acid was taken as a control compound. A blank control group (without ethanol) and a model group (with ethanol but without the salts of the examples or glycyrrhetinic acid) were set.

GES-1 human gastric mucosal epithelial cells were uniformly inoculated into a 96-well plate, about 5,000 cells per well. The cells were cultured in a complete RPMI 1640 medium for 6 h. After the cells were completely adhered to the wall, all the media were removed, 100 µL of cell culture solution with serum-free RPMI 1640 medium containing 7% ethanol (a serum-free RPMI 1640 medium without ethanol was added to the blank group) was added to each well, and the cells were incubated and cultured for 12 h. After the cells were incubated in the ethanol for 12 h, the cell viability was significantly reduced to about 60%.

Then, all the media were removed again, 100 µL of each of serum-free RPMI 1640 media of cell culture solutions, containing 0.025 g/mL, 0.05 g/mL, 0.1 g/mL, 0.2 g/mL, 0.4 g/mL, and 0.8 g/mL experimental or control compounds (a serum-free RPMI 1640 medium without the compounds was added to the blank group and a model group), were added to each well according to the groups, and the cells were cultured for 24 h.

Then, the cell viability was detected. Results of the model group show that after the cells are cultured in a medium containing carbon tetrachloride for 12 h, the cell viability is significantly reduced to about 60%. Results of the experimental group and the control group are shown in Table 1. It can be seen that after the salt of the present application is added to the cell culture solution, the cell viability increases with the increase of concentration, and the cell viability in the groups of the salts of the present application is significantly higher than that of the glycyrrhetinic acid control group.

**Table 1 Cell viability of ethanol-induced damage model of gastric mucosal epithelial cells**

| Cell viability (%) | | Compound concentration (mg/mL) | | | | | |
|---|---|---|---|---|---|---|---|
| | | 0.025 | 0.5 | 0.1 | 0.2 | 0.4 | 0.8 |
| | Example 51 | 62.5 | 72.4 | 79.6 | 86.9 | 88.7 | 92.4 |
| Compound | Example 57 | 63.7 | 70.1 | 77.4 | 85.3 | 88.1 | 93.2 |
| | Glycyrrhetinic acid | 59.4 | 61.8 | 64.3 | 65.2 | 68.9 | 71.9 |

### Neurodegenerative disease - Parkinson's disease model

6-hydroxydopamine (6-OHDA) could be taken up by nerve cells, causing metabolic disorders of nerve cells and further inducing a Parkinson's disease model in vitro. In this experiment, a Parkinson's disease cell model was established at a cellular level with 6-OHDA, the salt of the present application or another control compound was added, and the technical effects of the non-covalent dimer cation of the present application in the Parkinson's disease cell model were obtained.

The salts of example 51 and examples 57 were taken as experimental compounds, and selegiline was taken as a control compound. A blank control group (without 6-OHDA) and a model group (with 6-OHDA but without the salts of the examples or selegiline) were set.

SH-SY5Y human neuroblastoma cells were uniformly inoculated into a 96-well plate, about 5,000 cells per well. The cells were cultured in a complete DMEM/F12 medium for 6 h. After the cells were completely adhered to the wall, all the media were removed, 100 µL of cell culture solution with serum-free DMEM/F12 medium containing 150 µmol/L 6-OHDA (a serum-free DMEM/F12 medium without 6-OHDA was added to the blank group) was added to each well, and the cells were incubated and cultured for 24 h. After the cells were incubated in 6-OHDA for 12 h, the cell viability was significantly reduced to about 30%.

Then, all the media were removed again, 100 µL of each of serum-free DMEM/F12 media of cell culture solutions, containing 0.025 g/mL, 0.05 g/mL, 0.1 g/mL, 0.2 g/mL, 0.4 g/mL, and 0.8 g/mL experimental or control compounds (a serum-free DMEM/F12 medium without the compounds was added to the blank group and a model group), were added to each well according to the groups, and the cells were cultured for 24 h.

Then, the cell viability was detected. Results of the model group show that after the cells are cultured in a medium containing 6-OHDA for 24 h, the cell viability is significantly reduced to about 30%. Results of the experimental group and the control group are shown in Table 2. It can be seen that after the salt of the present application is added to the cell culture solution, the cell viability increases with the increase of concentration, and the cell viability in the groups of the salts of the present application is significantly higher than that of the selegiline control group.

**Table 2 Cell viability of Parkinson's disease model**

| Cell viability (%) | | Compound concentration (mg/mL) | | | | | |
|---|---|---|---|---|---|---|---|
| | | 0.025 | 0.5 | 0.1 | 0.2 | 0.4 | 0.8 |
| Compound | Example 51 | 37.0 | 42.6 | 48.2 | 56.8 | 66.7 | 78.5 |
| | Example 57 | 42.3 | 56.2 | 68.5 | 78.8 | 86.7 | 95.7 |
| | Selegiline | 33.4 | 37.5 | 43.3 | 49.9 | 58.5 | 69.8 |

### Neurodegenerative disease - Alzheimer's disease model

β amyloid protein (Aβ) is often present in hippocampal neurons in Alzheimer's disease, causing hippocampal neuron damage, and thus leading to Alzheimer's disease. Therefore, in this experiment, an Alzheimer's disease cell model was established at a cellular level with Aβ(1-42) as one of subtypes of Aβ, the salt of the present application or another control compound was added, and the technical effects of the non-covalent dimer cation of the present application in the Alzheimer's disease cell model were obtained.

The salts of example 51 and example 57 were taken as experimental compounds, and ganglioside was taken as a control compound. A blank control group (without Aβ(1-42)) and a model group (with Aβ(1-42) but without the salts of the examples or ganglioside) were set.

SH-SY5Y human neuroblastoma cells were uniformly inoculated into a 96-well plate, about 5,000 cells per well. The cells were cultured in a complete DMEM/F12 medium for 6 h. After the cells were completely adhered to the wall, all the media were removed, 100 µL of cell culture solution with serum-free DMEM/F12 medium containing 20 µmol/L Aβ(1-42) (a serum-free DMEM/F12 medium without Aβ(1-42) was added to the blank group) was added to each well, and the cells were incubated and cultured for 24 h. After the cells were incubated in Aβ(1-42) for 48 h, the cell viability was significantly reduced to about 50%.

Then, all the media were removed again, 100 µL of each of serum-free DMEM/F12 media of cell culture solutions, containing 0.025 g/mL, 0.05 g/mL, 0.1 g/mL, 0.2 g/mL, 0.4 g/mL, and 0.8 g/mL experimental or control compounds (a serum-free DMEM/F12 medium without the compounds was added to the blank group and a model group), were added to each well according to the groups, and the cells were cultured for 24 h.

Then, the cell viability was detected. Results of the model group show that after the cells are cultured in a medium containing Aβ(1-42) for 48 h, the cell viability is significantly reduced to about 50%. Results of the experimental group and the control group are shown in Table 3. It can be seen that after the salt of the present application is added to the cell culture solution, the cell viability increases with the increase of concentration, and the cell viability in the groups of the salts of the present application is significantly higher than that of the ganglioside control group.

**Table 3 Cell viability of Alzheimer's disease model**

| Cell viability (%) | | Compound concentration (mg/mL) | | | | | |
|---|---|---|---|---|---|---|---|
| | | 0.025 | 0.5 | 0.1 | 0.2 | 0.4 | 0.8 |
| Compound | Example 51 | 45.3 | 63.5 | 70.6 | 78.8 | 85.5 | 92.6 |
| | Example 57 | 52.3 | 67.2 | 76.8 | 83.6 | 90.5 | 97.7 |
| | Ganglioside | 46.4 | 50.3 | 58.0 | 63.2 | 66.3 | 73.5 |

The above embodiments are only the preferred embodiments of the present application and cannot be intended to limit the protection scope of the present application, and any non-substantial changes and substitutions made by a person skilled in the art based on the present application fall within the protection scope of the present application.

## Claims

1. A non-covalent dimer cation, comprising or consisting of a cation of formula [⁺Q¹-H···Q²], a tautomer thereof, or a stereoisomer thereof,
wherein a bond "···" in formula [⁺Q¹-H···Q²] is a non-covalent bond;
one of Q¹ and Q² is a first moiety, and the other is a second moiety;
the first moiety is selected from a group consisting of formula (R), formula (Y), and dihydro derivatives thereof, and the second moiety is formula (A), 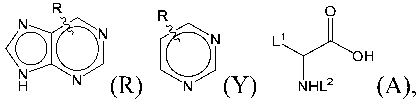
wherein R is selected from a group consisting of no group, -H, -CH₃, =CH₂, -NH₂, =NH, - OH, =O, -F, β-D-ribofuranosyl, and 2-deoxy-β-D-ribofuranosyl; and
-L² is H, and -L¹ is selected from a group consisting of hydrogen, methyl, hydroxymethyl, mercaptomethyl, selenomethyl, aminomethyl, carboxymethyl, carbamoylmethyl, methylthiomethyl, guanidinomethyl, ethyl, 1-hydroxyethyl, 2-hydroxyethyl, 1-mercaptoethyl, 2-mercaptoethyl, 1-aminoethyl, 2-aminoethyl, 1-carboxyethyl, 2-carboxyethyl, 1-carbamoylethyl, 2-carbamoylethyl, 1-methylthioethyl, 2-methylthioethyl, 1-guanidinoethyl, 2-guanidinoethyl, n-propyl, isopropyl, 1-hydroxypropyl, 2-hydroxypropyl, 3-hydroxypropyl, 1-mercaptopropyl, 2-mercaptopropyl, 3-mercaptopropyl, 1-aminopropyl, 2-aminopropyl, 3-aminopropyl, 1-carboxypropyl, 2-carboxypropyl, 3-carboxypropyl, 1-carbamoylpropyl, 2-carbamoylpropyl, 3-carbamoylpropyl, 1-methylthiopropyl, 2-methylthiopropyl, 3-methylthiopropyl, 1-guanidinopropyl, 2-guanidinopropyl, 3-guanidinopropyl, n-butyl, isobutyl, sec-butyl, tert-butyl, 4-aminobutyl, 1-hydroxybutyl, 2-hydroxybutyl, 3-hydroxybutyl, 4-hydroxybutyl, 1-mercaptobutyl, 2-mercaptobutyl, 3-mercaptobutyl, 4-mercaptobutyl, 1-aminobutyl, 2-aminobutyl, 3-aminobutyl, 4-aminobutyl, 1-carboxybutyl, 2-carboxybutyl, 3-carboxybutyl, 4-carboxybutyl, 1-carbamoylbutyl, 2-carbamoylbutyl, 3-carbamoylbutyl, 4-carbamoylbutyl, 1-methylthiobutyl, 2-methylthiobutyl, 3-methylthiobutyl, 4-methylthiobutyl, 1-guanidinobutyl, 2-guanidinobutyl, 3-guanidinobutyl, 4-guanidinobutyl, benzyl, 2-hydroxybenzyl, 3-hydroxybenzyl, 4-hydroxybenzyl, (pyrrol-4-yl)methyl, (indol-3-yl)methyl, and (3-methyl-3,4-dihydropyrrole-2-carbonyl)aminobutyl; or -L¹ and -L² together form a group selected from a group consisting of propane-1,3-diyl, butane-1,4-diyl, and pentane-1,5-diyl.

2. The cation according to claim 1, wherein
the first moiety is selected from a group consisting of formula (R'), formula (Y'), and dihydro derivatives thereof, 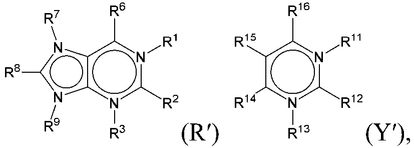
wherein R¹, R², R³, R⁶, R⁷, R⁸, R⁹, R¹¹, R¹², R¹³, R¹⁴, R¹⁵*,* and R¹⁶ are each independently selected from a group consisting of no group, -X¹, and =X², and particularly, selected from the group consisting of no group, -H, -CH₃, =CH₂, -NH₂, =NH, -OH, =O, -F, β-D-ribofuranosyl, and 2-deoxy-β-D-ribofuranosyl.

3. The cation according to claim 1 or 2, wherein
the first moiety is selected from a group consisting of formula (Ra), formula (Rb), formula (Ya), and formula (Yb), 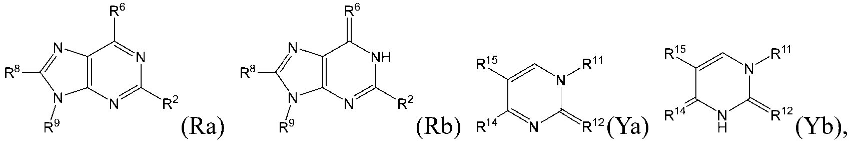 wherein
-R², -R⁸, -R⁹, -R¹¹, and -R¹⁵ are each independently selected from a group consisting of -H, -CH₃, -NH₂, -OH, -F, and β-D-ribofuranosyl;
=R¹² is selected from a group consisting of =CH₂, =NH, and =O;
in formula (Ra), -R⁶ is selected from a group consisting of -H, -CH₃, -NH₂, -OH, - F, β-D-ribofuranosyl, and 2-deoxy-β-D-ribofuranosyl, and in formula (Rb), =R⁶ is selected from the group consisting of =CH₂, =NH, and =O; and
in formula (Ya), -R¹⁴ is selected from the group consisting of -H, -CH₃, -NH₂, -OH, -F, β-D-ribofuranosyl, and 2-deoxy-β-D-ribofuranosyl, and in formula (Yb), =R¹⁴ is selected from the group consisting of =CH₂, =NH, and =O.
-L² is H, and -L¹ is selected from a group consisting of hydrogen, methyl, hydroxymethyl, mercaptomethyl, selenomethyl, carboxymethyl, carbamoylmethyl, 1-hydroxyethyl, 2-carboxyethyl, 2-carbamoylethyl, 2-methylthioethyl, isopropyl, 3-guanidinopropyl, isobutyl, sec-butyl, 4-aminobutyl, benzyl, 4-hydroxybenzyl, (pyrrol-4-yl)methyl, (indol-3-yl)methyl, and (3-methyl-3,4-dihydropyrrole-2-carbonyl)aminobutyl; or -L¹ and -L² together form propane-1,3-diyl.

4. The cation according to any one of claims 1 to 3, wherein
the first moiety and the Q² moiety is selected from a group consisting of 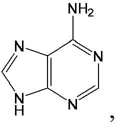 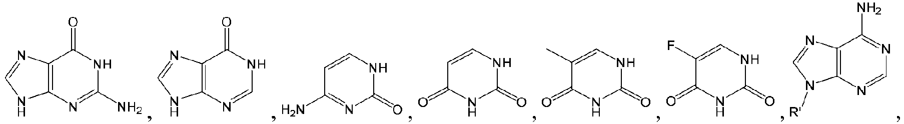 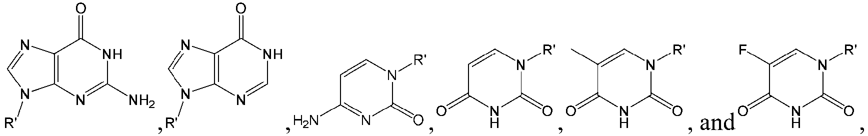 wherein R' is β-D-ribofuranosyl; and
the second moiety is selected from a group consisting of alanine, asparagine, histidine, proline, pyrrolysine, serine, tryptophan, and tyrosine.

5. A salt, comprising:
the cation according to any one of claims 1 to 4; and
a first anion,
wherein the first anion comprises at least one selected from a group consisting of a fluoride ion, a chloride ion, a bromide ion, an iodide ion, a sulfide ion, a nitrate ion, a sulfate ion, a sulfite ion, a thiosulfate ion, a persulfate ion, a selenate ion, a phosphate ion, a carbonate ion, a hexafluorophosphate ion, a hexafluorosilicate ion, an acetate ion, a succinate ion, a sulfonate ion, a benzoate ion, and a polyphosphate ion.

6. The salt according to claim 5, wherein the first anion comprises at least one selected from a group consisting of a hexafluorophosphate ion, an acetate ion, a succinate ion, a sulfate ion, a phosphate ion, and a chloride ion.

7. A method of preparing the cation according to any one of claims 1 to 4 or the salt according to claim 5 or 6, comprising:
contacting a first reactant with a first acid to obtain a first acid salt of the first reactant;
contacting the first acid salt of the first reactant with a first salt comprising a first anion in a first acidic environment, to obtain a first solution comprising a protonated first acid salt cation of the first reactant and the first anion;
adding a second reactant to the first solution in a second acidic environment, to obtain a second solution or a first intermediate; and
adding a first base to the second solution or contacting the first base with the first intermediate,
wherein the first reactant comprises a compound of formula Q¹, and the second reactant comprises a compound of formula Q².

8. The method according to claim 7, wherein
the first acid is selected from a group consisting of hydrochloric acid, hydrobromic acid, and hydroiodic acid; and
the first salt consists of a first cation and the first anion, and the first cation is an ammonium ion;
and/or
the contacting a first reactant with a first acid is performed at a first temperature, and the first temperature is 0°C to 70°C;
the contacting the first acid salt of the first reactant with a first salt is performed at a second temperature, and the second temperature is 0°C to 70°C; and
the adding a second reactant to the first solution is performed at a third temperature, and the third temperature is 0°C to 70°C;
and/or
a molar ratio of the first reactant to the first acid is 1: 2 to 1: 10;
a molar ratio of the first acid salt of the first reactant to the first salt is 1: 2 to 1: 10; and
a molar ratio of a protonated first reactant cation to the second reactant is 1: 1 to 1: 2;
and/or
the first acidic environment has a pH of less than or equal to 6; and
the second acidic environment has a pH of less than or equal to 6;
and/or
the first base comprises at least one selected from a group consisting of ammonia water, sodium hydroxide, potassium hydroxide, and triethanolamine; and
the adding a first base to the second solution or contacting the first base with the first intermediate refers to adjusting the pH of the second solution or a solution comprising the first intermediate to 8 or more.

9. Use of the cation according to any one of claims 1 to 4, the salt according to claim 5 or 6, or the cation or salt prepared by the method according to claim 7 or 8 in antioxidation, preparation of an antioxidant, preparation of a medicament for inhibiting, reducing, or reversing oxidative stress in human or animal cells or for inhibiting, reducing, or scavenging oxygen species in human or animal bodies, or preparation of a medicament for treating an oxidative stress- or oxygen species-associated disease or symptom;
optionally, the disease or symptom is selected from a group consisting of ageing, inflammation, overweight, obesity, cancer, tumor, hepatopathy, neurodegenerative disease, arterial hypertension, atherosclerosis, cardiovascular disease, diabetes, hypercholesterolemia, chronic fatigue syndrome, ischemia-reperfusion damage, ultraviolet-induced damage, and alcohol-induced damage;
optionally, the disease or symptom is Parkinson's disease or Alzheimer's disease;
optionally, the disease or symptom is mucosal damage.

10. Use of the cation according to any one of claims 1 to 4, the salt according to claim 5 or 6, or the cation or salt prepared by the method according to claim 7 or 8 in promotion of cell proliferation, or preparation of a medicament for promoting cell proliferation or treating, alleviating, or repairing wounds;
optionally, the cells are cells located in the skin;
optionally, the cells are selected from a group consisting of epidermal fibroblasts, keratinocytes, vascular endothelial cells, and nerve cells;
optionally, the wounds comprise a skin wound.
